# DEMANDE DE BREVET EUROPEEN

(11) **EP 2 305 208 A2**
(43) Date de publication de la demande: **06.04.2011**
(21) Numéro de dépôt: 09179304.2
(22) Date de dépôt: 15.12.2009
(51) Int. Cl.: A61K 8/81, A61K 8/85, A61Q 5/06

(54) **Composition cosmétique comprenant un polyester sulfonique ramifié et un épaississant particulier et utilisations en coiffage**

(30) Priorité: 17.12.2008 FR 0858673
(71) Demandeur: L'Oréal, 75008 Paris (FR)
(72) Inventeur: Pasquet, Dorothée, 92270, Bois-Colombes (FR); Smail, Nadia, 78480, Verneuil sur Seine (FR); Gawtrey, Jonathan, 92100, Boulogne (FR); Bebot, Cécile, 92110, Clichy (FR)
(74) Mandataire: Dossmann, Gérard

(57) **Abrégé**

La présente demande concerne une composition cosmétique comprenant, dans un milieu cosmétiquement acceptable :
(i) un ou plusieurs polyesters sulfoniques ramifiés et
(ii) un ou plusieurs polymères épaississants (méth)acryliques et
(iii) un ou plusieurs tensioactifs choisis parmi les tensioactifs anioniques, non ioniques, amphotères ou cationiques.

La présente demande concerne aussi les utilisations de cette composition notamment pour le coiffage et la mise en forme des cheveux.

## Description

La présente invention concerne de nouvelles compositions cosmétiques comprenant un ou plusieurs polyesters sulfoniques ramifiés, un ou plusieurs polymères épaississants (méth)acryliques et un ou plusieurs tensioactifs choisis parmi les tensioactifs anioniques, non ioniques, amphotères ou cationiques, ainsi que les utilisations de ces compositions notamment en coiffage.

La présente invention concerne aussi un procédé de coiffage des matières kératiniques mettant en oeuvre ces compositions.

L'utilisation de polyesters sulfoniques ramifiés dans des compositions de coiffage et de fixation des cheveux est connue et décrite par exemple dans les demandes de brevet EP 0 966 946, WO 98/38969 et WO 99/63955.

Néanmoins l'utilisation des polyesters sulfoniques ramifiés n'est pas sans inconvénients :
■ l'utilisation de ces polyesters dans des laques capillaires contenant des quantités d'alcool importantes donne généralement de bonnes propriétés coiffantes mais ne permet pas d'obtenir un pouvoir laquant satisfaisant ;
■ l'application de ces polyesters sous forme de laques à forte teneur en alcool confère aux cheveux, après brossage, un toucher sec ; ce phénomène indésirable est particulièrement visible pour les cheveux teints ;
■ ces polyesters se présentent généralement sous forme semisolide, cette propriété rend leur mise en oeuvre souvent difficile, en particulier, il est souvent difficile d'assurer une répartition homogène de ces polyesters sur toute la chevelure à traiter.

De manière inattendue et avantageuse, la Demanderesse a mis en évidence que l'utilisation d'un ou plusieurs polyesters sulfoniques ramifiés avec un ou plusieurs polymères épaississants (méth)acryliques et un ou plusieurs tensioactifs permet de pallier aux inconvénients précités.

La présente invention a ainsi pour objet une composition cosmétique comprenant un ou plusieurs polyesters sulfoniques ramifiés, un ou plusieurs polymères épaississants (méth)acryliques et un ou plusieurs tensioactifs choisis parmi les tensioactifs anioniques, non ioniques, amphotères ou cationiques.

Par « polymères épaississants (méth)acryliques » au sens de la présente demande, on entend des polymères (méth)acryliques épaississants non fixants.

Les compositions obtenues se présentent sous la forme de gels, mousses, sprays, crèmes, pâtes.

Les compositions selon la présente invention sont faciles à préparer et à appliquer. Elles restent bien localisées sans coulures au point d'application. Les compositions selon la présente invention peuvent être appliquées sans chute de viscosité dans le temps.

En outre, les compositions selon la présente invention permettent de conférer à la coiffure un maintien naturel et durable.

La présente invention a encore pour objet un procédé de coiffage des matières kératiniques, de préférence les matières kératiniques humaines et en particulier les cheveux mettant en oeuvre les compositions selon l'invention.

La présente invention a également pour objet les utilisations des compositions selon l'invention notamment pour le coiffage, la mise en forme des matières kératiniques, de préférence les matières kératiniques humaines et en particulier les cheveux.

D'autres caractéristiques, aspects, objets et avantages de l'invention apparaîtront encore plus clairement à la lecture de la description et des exemples qui suivent.

Par « (méth)acrylique » au sens de la présente demande on entend « acrylique ou méthacrylique » .

Les polyesters sulfoniques ramifiés utilisés dans les compositions de la présente invention sont connus dans l'art antérieur. Leur structure et synthèse sont décrites dans les documents WO 95/18191, WO 97/08261 et WO 97/20899.

On préfère en particulier des polyesters sulfoniques ramifiés obtenus par polycondensation :
(a) d'au moins un acide dicarboxylique ne portant pas de fonction sulfonique,
(b) d'au moins un diol ou d'un mélange d'un diol et d'une diamine,
(c) d'au moins un monomère comportant deux fonctions réactives, identiques ou différentes, choisies parmi les groupes hydroxyle, amino et carboxyle, et portant en outre au moins une fonction sulfonique, et
(d) d'au moins un monomère comportant au moins trois fonctions réactives, identiques ou différentes, choisies parmi les groupes hydroxyle, amino et carboxyle.

Les acides dicarboxyliques formant les motifs (a) peuvent être des acides dicarboxyliques aliphatiques, des acides dicarboxyliques alicycliques, des acides dicarboxyliques aromatiques et des mélanges de tels acides.

On peut citer à titre d'exemples l'acide 1,4-cyclohexanedioïque, l'acide succinique, l'acide glutarique, l'acide adipique, l'acide azélaïque, l'acide sébacique, l'acide fumarique, l'acide maléique, l'acide 1,3-cyclohexanedioïque, l'acide phtalique, l'acide téréphtalique et l'acide isophtalique et les mélanges de tels acides.

Les diols formant les motifs (b) sont choisis par exemple parmi les alcanediols et les polyalkylènediols, et on peut citer à titre d'exemple l'éthylèneglycol, le propylèneglycol, le diéthylèneglycol, le triéthylèneglycol et le polypropylèneglycol.

Les diamines susceptibles de former une partie des motifs (b) sont de préférence choisies parmi les alcanediamines et les poly(oxyalkylène)diamines.

Le terme « fonction sulfonique » des motifs (c) englobe à la fois la fonction acide sulfonique (-SO₃H) et les fonctions salifiées correspondantes obtenues par neutralisation de la fonction acide sulfonique avec une base, par exemple un hydroxyde de métal alcalin.

Les fonctions sulfoniques sont de préférence sous forme neutralisée par une base organique ou minérale.

Les motifs (c) sont dérivés par exemple d'acides dicarboxyliques, d'esters d'acides dicarboxyliques, de glycols et d'hydroxyacides, portant tous au moins un groupe sulfonique, sous forme acide et/ou neutralisée, de préférence sous forme neutralisée.

Les motifs (c) portant au moins une fonction sulfonique représentent de préférence de 2 à 15% en moles de l'ensemble des monomères.

Les motifs (d) dérivés de monomères multifonctionnels sont présents de préférence en une quantité comprise entre 0,1 et 40% en moles rapportée à l'ensemble des monomères.

Les monomères multifonctionnels formant les motifs (d) sont choisis par exemple parmi le triméthyloléthane, le triméthylolpropane, le glycérol, le pentaérythritol, le sorbitol, l'anhydride trimellitique, l'érythritol, le thréitol, le dipentaérythritol, le dianhydride pyromellitique et l'acide diméthylpropionique.

Les polyesters sulfoniques ramifiés peuvent comporter, en plus des quatre types de motifs (a) à (d) décrits ci-dessus, des motifs (e) dérivés de monomères comportant deux fonctions réactives différentes, choisis par exemple parmi les hydroxyacides carboxyliques et les aminoacides carboxyliques ou les mélange de ceux-ci.

Ces motifs (e) peuvent représenter jusqu'à 40% en moles de l'ensemble des monomères (a), (b), (c), (d) et (e).

Bien entendu, les polymères sulfoniques ramifiés utilisés dans la présente invention sont obtenus de préférence à partir d'un mélange de monomères dans lequel le nombre d'équivalents de fonctions acide carboxylique est substantiellement égal au nombre d'équivalents de fonctions hydroxyle et de fonctions amino, éventuellement présentes.

Les polymères sulfoniques ramifiés utilisés dans les compositions de coiffage de la présente invention sont connus et commercialisés par exemple par la société Eastman. On peut citer à titre de produit commercial préféré le produit vendu sous la dénomination AQ 1350^{®} par la société Eastman.

La composition selon la présente invention comprend avantageusement de 0,2 à 15%, de préférence de 0,5 à 10% et de manière encore plus préférée de 1 à 10% en poids d'un ou plusieurs polyesters sulfoniques ramifiés par rapport au poids total de la composition.

La composition selon la présente invention contient en outre un ou plusieurs polymères épaississants (méth)acryliques.

Par polymère acrylique on entend au sens de la présente invention un polymère résultant de la polymérisation d'au moins un ou plusieurs monomères de structure : 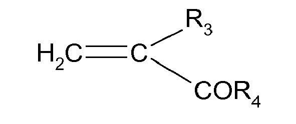
R3 désignant un atome d'hydrogène, un radical alkyl en C₁-C₄ linéaire ou ramifié,
R4 désignant un atome d'hydrogène, un radical alkyl en C₁-C₄ linéaire ou ramifié, un radical NR5R6, un radical alcoxy en C₁-C₃₀ linéaire ou ramifié, éventuellement substitué par un ou plusieurs radicaux hydroxyle, par un radical ammonium quaternaire,
R5 et R6 désignent un atome d'hydrogène, un radical alkyle en C₁-C₃₀ éventuellement oxyalkyléné, le radical alkyle pouvant comporter un groupement sulfonique.

De préférence R3 désigne un atome d'hydrogène ou un radical méthyle.

Par polymère épaississant, on entend au sens de la présente invention un polymère capable par sa présence d'augmenter d'au moins 50 centipoises à 25°C et à un taux de cisaillement de 1 s⁻¹ la viscosité du milieu. De préférence, le polymère épaississant à 1% dans l'eau ou un mélange eau/alcool 50/50 en poids présente une viscosité à 25°C et à un taux de cisaillement de 1 s⁻¹ supérieure à 100 centipoises. Ces viscosités peuvent être mesurées à l'aide notamment de viscosimètres ou rhéomètres à géométrie cône plan.

Les polymères épaississants acryliques sont notamment choisis parmi :
(a) les épaississants associatifs acryliques;
(b) les homopolymères d'acide acrylique réticulés ;
(c) les copolymères réticulés d'acide (méth)acrylique et d'acrylate d'alkyle(C₁-C₆),
(d) les homopolymères et copolymères non-ioniques contenant des monomères à insaturation éthylénique de type ester et/ou amide ;
(e) les homopolymères d'acrylate d'ammonium ou les copolymères d'acrylate d'ammonium et d'acrylamide ;
(f) les homopolymères et copolymères d'acides (méth)acrylamido alkyl(C₁-C₄) sulfoniques,
(g) les homopolymères et copolymères de méthacryoyl alkyl(C₁-C₄) trialkyl(C1-C4) ammonium réticulés.

Par l'expression "épaississant associatif", on entend selon l'invention, un épaississant amphiphile comportant à la fois des motifs hydrophiles et des motifs hydrophobes en particulier comportant au moins une chaîne grasse en C8-C30 et au moins un motif hydrophile.

On peut utiliser comme épaississants associatifs acryliques selon l'invention les polymères associatifs acryliques choisis parmi :
(i) les polymères amphiphiles non-ioniques comportant au moins une chaîne grasse et au moins un motif hydrophile ;
(ii) les polymères amphiphiles anioniques comportant au moins un motif hydrophile, et au moins un motif à chaîne grasse ;
(iii) les polymères amphiphiles cationiques comportant au moins un motif hydrophile, et au moins un motif à chaîne grasse ;
(iv) les polymères amphiphiles amphotères comportant au moins un motif hydrophile, et au moins un motif à chaîne grasse ;
les chaînes grasses ayant de 10 à 30 atomes de carbone.
(i) Les polymères amphiphiles non-ioniques acryliques comportant au moins une chaîne grasse et au moins un motif hydrophile sont choisis de préférence parmi :
   (1) les copolymères de méthacrylates ou d'acrylates d'alkyles en C1-C6 et de monomères amphiphiles comportant au moins une chaîne grasse (par exemple les acrylates d'alkyl (C₈-C₂₂) oxyéthyléné) tels que par exemple le copolymère méthacrylate de méthyle/acrylate de stéaryle oxyéthyléné vendu par la société GOLDSCHMIDT sous la dénomination ANTIL 208.
   (2) les copolymères de méthacrylates ou d'acrylates hydrophiles et de monomères hydrophobes comportant au moins une chaîne grasse (par exemple les (meth)acrylates d'alkyl (C₈-C₂₂)) tels que par exemple le copolymère méthacrylate de polyéthylèneglycol/méthacrylate de lauryle.
(ii) Les polymères amphiphiles anioniques acryliques peuvent être choisis parmi ceux comportant au moins un motif hydrophile de type acide carboxylique insaturé oléfinique, et au moins un motif hydrophobe de type ester d'alkyl (C₁₀-C₃₀) d'acide carboxylique insaturé. Ils sont choisis de préférence parmi ceux dont le motif hydrophile de type acide carboxylique insaturé oléfinique correspond au monomère de formule (I) suivante : 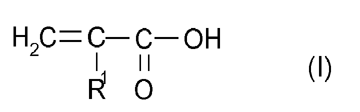 formule dans laquelle, R1 désigne H ou CH₃ ou C₂H₅, c'est-à-dire des motifs acide acrylique, acide méthacrylique ou acide éthacrylique et dont le motif hydrophobe de type ester d'alkyl (C₁₀-C₃₀) d'acide carboxylique insaturé correspond au monomère de formule (II) suivante : 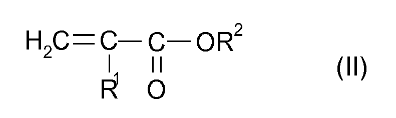 formule dans laquelle, R1 désigne H ou CH₃ ou C₂H₅ (c'est-à-dire des motifs acrylates, méthacrylates ou éthacrylates) et de préférence H (motifs acrylates) ou CH3 (motifs méthacrylates), R2 désignant un radical alkyle en C₁₀-C₃₀, et de préférence en C₁₂-C₂₂.

Des esters d'alkyls (C₁₀-C₃₀) d'acides carboxyliques insaturés conformes à l'invention comprennent par exemple, l'acrylate de lauryle, l'acrylate de stéaryle, l'acrylate de décyle, l'acrylate d'isodécyle, l'acrylate de dodécyle, et les méthacrylates correspondants, le méthacrylate de lauryle, le méthacrylate de stéaryle, le méthacrylate de décyle, le méthacrylate d'isodécyle, et le méthacrylate de dodécyle.

Des polymères amphiphiles anioniques de ce type sont par exemple décrits et préparés, selon les brevets US-3 915 921 et 4 509 949.

Les polymères amphiphiles anioniques utilisables dans le cadre de la présente invention peuvent désigner plus particulièrement des polymères formés à partir d'un mélange de monomères comprenant :
(i) de l'acide acrylique et un ou plusieurs esters de formule (III) suivante : 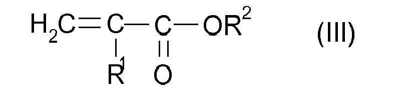 dans laquelle R1 désigne H ou CH3, R2 désignant un radical alkyle ayant de 12 à 22 atomes de carbone, et un agent réticulant, tels que par exemple ceux constitués de 95 à 60% en poids d'acide acrylique (motif hydrophile), 4 à 40% en poids d'acrylate d'alkyles en C10-C30 (motif hydrophobe), et 0 à 6% en poids de monomère polymérisable réticulant, ou 98 à 96% en poids d'acide acrylique (motif hydrophile), 1 à 4% en poids d'acrylate d'alkyles en C10-C30 (motif hydrophobe), et 0,1 à 0,6% en poids de monomère polymérisable réticulant,
(ii) essentiellement de l'acide acrylique et du méthacrylate de lauryle tel que celui formé à partir de 66% en poids d'acide acrylique et 34% en poids de méthacrylate de lauryle.

Ledit réticulant est un monomère contenant un groupe 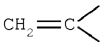 avec au moins un autre groupement polymérisable dont les liaisons insaturées sont non conjuguées l'une par rapport à l'autre. On peut notamment citer les polyallyléthers tels que notamment le polyallylsucrose et le polyallylpentaérythritol.

Parmi lesdits polymères ci-dessus, on préfère tout particulièrement selon la présente invention, les produits vendus par la société GOODRICH sous les dénominations commerciales PEMULEN TR1, PEMULEN TR2, CARBOPOL 1382, et encore plus préférentiellement le PEMULEN TR1, et le produit vendu par la société S.E.P.C. sous la dénomination COATEX SX.

Comme polymères amphiphiles anioniques à chaînes grasses, on peut également citer le copolymère acide méthacrylique/acrylate de méthyle/diméthylméta- isopropényl benzyl isocyanate d'alcool éthoxylé commercialisé sous la dénomination VISCOPHOBE DB 1000 par la société AMERCHOL.

Comme autres polymères amphiphiles anioniques à chaînes grasses, on peut citer ceux comportant au moins un monomère acrylique à groupement(s) sulfonique(s), sous forme libre ou partiellement ou totalement neutralisée et comprenant au moins une partie hydrophobe.

La partie hydrophobe présente dans les polymères de l'invention comporte de préférence de 8 à 22 atomes de carbone, plus préférentiellement encore de 8 à 18 atomes de carbone, et plus particulièrement de 12 à 18 atomes de carbone.

De façon préférentielle, les polymères sulfoniques conformes à l'invention sont neutralisés partiellement ou totalement par une base minérale (soude, potasse, ammoniaque) ou une base organique telle que la mono-, di- ou tri-éthanolamine, un aminométhylpropanediol, la N-méthyl-glucamine, les acides aminés basiques comme l'arginine et la lysine, et les mélanges de ces composés.

Les polymères amphiphiles sulfoniques conformes à l'invention ont généralement un poids moléculaire moyen en nombre allant de 1000 à 20 000 000 g/mole, de préférence allant de 20 000 à 5 000 000 et plus préférentiellement encore de 100 000 à 1 500 000 g/mole.

Les polymères amphiphiles sulfoniques selon l'invention peuvent être réticulés ou non-réticulés. De préférence, on choisit des polymères amphiphiles réticulés.

Lorsqu'ils sont réticulés, les agents de réticulation peuvent être choisis parmi les composés à polyinsaturation oléfinique couramment utilisés pour la réticulation des polymères obtenus par polymérisation radicalaire. On peut citer par exemple, le divinylbenzène, l'éther diallylique, le dipropylèneglycol-diallyléther, les polyglycol-diallyléthers, le triéthylèneglycol-divinyléther, l'hydroquinone-diallyl-éther, le di(méth)acrylate de d'éthylèneglycol ou de tétraéthylèneglycol, le triméthylol propane triacrylate, le méthylène-bis-acrylamide, le méthylène-bis-méthacrylamide, la triallylamine, le triallylcyanurate, le diallylmaléate, la tétraallyléthylènediamine, le tétra-allyloxy-éthane, le triméthylolpropane-diallyléther, le (méth)acrylate d'allyle, les éthers allyliques d'alcools de la série des sucres, ou d'autres allyl- ou vinyl- éthers d'alcools polyfonctionnels, ainsi que les esters allyliques des dérivés de l'acide phosphorique et/ou vinylphosphonique, ou les mélanges de ces composés.

On utilisera plus particulièrement le méthylène-bis-acrylamide, le méthacrylate d'allyle ou le triméthylol propane triacrylate (TMPTA). Le taux de réticulation variera en général de 0,01 à 10% en mole et plus particulièrement de 0,2 à 2% en mole par rapport au polymère.

Les monomères acryliques à groupement(s) sulfonique(s) sont choisis notamment parmi les acides (méth)acrylamido(C₁-C₂₂)alkylsulfoniques, les acides N-(C1-C22)alkyl(méth)acrylamido(C₁-C₂₂)alkylsulfoniques comme l'acide undécyl-acrylamido-méthane-sulfonique ainsi que leurs formes partiellement ou totalement neutralisées.

Plus préférentiellement, on utilisera les acides (méth)acrylamido(C₁-C₂₂) alkylsulfoniques tels que par exemple l'acide acrylamido-méthane-sulfonique, l'acide acrylamido-éthane-sulfonique, l'acide acrylamido-propane-sulfonique, l'acide 2-acrylamido-2-méthylpropane-sulfonique, l'acide méthacrylamido-2-méthylpropane-sulfonique, l'acide 2-acrylamido-n-butane-sulfonique, l'acide 2-acrylamido-2,4,4-triméthylpentane-sulfonique, l'acide 2-méthacrylamido-dodécyl-sulfonique, l'acide 2-acrylamido-2,6-diméthyl-3-heptane-sulfonique ainsi que leurs formes partiellement ou totalement neutralisées.

Plus particulièrement, on utilisera l'acide 2-acrylamido-2-méthylpropane-sulfonique (AMPS) ainsi que ses formes partiellement ou totalement neutralisées.

Les polymères amphiphiles conformes à l'invention peuvent notamment être choisis parmi les polymères amphiphiles statistiques d'AMPS modifiés par réaction avec une n-monoalkylamine ou une di-n-alkylamine en C6-C22, et tels que ceux décrits dans la demande de brevet WO00/31154, les polymères décrit dans cette demande font partie du contenu de la présente description. Ces polymères peuvent également contenir d'autres monomères hydrophiles éthyléniquement insaturés choisis par exemple parmi les acides (méth)acryliques, leurs dérivés alkyl substitués en β ou leurs esters obtenus avec des monoalcools ou des mono- ou poly- alkylèneglycols, les (méth)acrylamides, la vinylpyrrolidone, l'anhydride maléique, l'acide itaconique ou l'acide maléique ou les mélanges de ces composés.

Les polymères de l'invention peuvent être choisis parmi les copolymères amphiphiles d'AMPS et d'au moins un monomère hydrophobe à insaturation éthylénique comportant au moins une partie hydrophobe ayant de 8 à 50 atomes de carbone et plus préférentiellement de 8 à 22 atomes de carbone et encore plus préférentiellement de 8 à 18 atomes de carbone et plus particulièrement 12 à 18 atomes de carbone.

Ces mêmes copolymères peuvent contenir en outre un ou plusieurs monomères éthyléniquement insaturés ne comportant pas de chaîne grasse tels que les acides (méth)acryliques, leurs dérivés alkyl substitués en β ou leurs esters obtenus avec des monoalcools ou des mono- ou poly- alkylèneglycols, les (méth)acrylamides, la vinylpyrrolidone, l'anhydride maléique, l'acide itaconique ou l'acide maléique ou les mélanges de ces composés.

Ces copolymères sont décrits notamment dans la demande de brevet EP-A-750899, le brevet US 5089578 et dans les publications de Yotaro Morishima suivantes :
« Self-assembling amphiphilic polyelectrolytes and their nanostructures - Chinese Journal of Polymer Science Vol. 18, N°40, (2000), 323-336. »
« Miscelle formation of random copolymers of sodium 2-(acrylamido)-2-methylpropanesulfonate and a non-ionic surfactant macromonomer in water as studied by fluorescence and dynamic light scattering - Macromolecules 2000, Vol. 33, N° 10 - 3694-3704 » ;
« Solution properties of miscelle networks formed by non-ionic moieties covalently bound to an polyelectrolyte : salt effects on rheological behavior - Langmuir, 2000, Vol.16, N°12, 5324-5332 » ;
« Stimuli responsive amphiphilic copolymers of sodium 2-(acrylamido)-2-methylpropanesulfonate and associative macromonomers - Polym. Preprint, Div. Polym. Chem. 1999, 40(2), 220-221».

Les monomères hydrophobes à insaturation éthylénique de ces copolymères particuliers sont choisis de préférence parmi les acrylates ou les acrylamides de formule (IV) suivante : 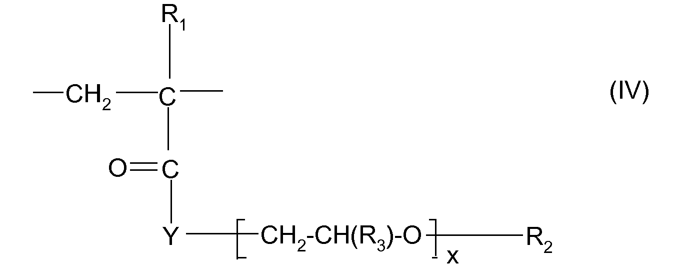 dans laquelle R1 et R3, identiques ou différents, désignent un atome d'hydrogène ou un radical alkyle linéaire ou ramifié en C1-C6 (de préférence méthyle) ; Y désigne O ou NH ; R2 désigne un radical hydrocarboné hydrophobe comportant au moins de 8 à 50 atomes de carbone et plus préférentiellement de 8 à 22 atomes de carbone et encore plus préférentiellement de 6 à 18 atomes de carbone et plus particulièrement de 12 à 18 atomes de carbone ; x désigne un nombre de moles d'oxyde d'alkylène et varie de 0 à 100.

Le radical R₂ est choisi de préférence parmi les radicaux alkyles en C₆-C₁₈, linéaires (par exemple n-hexyle, n-octyle, n-décyle, n-hexadécyle, n-dodécyle), ramifiés ou cycliques (par exemple cyclododécane (C₁₂) ou adamantane (C₁₀)) ; les radicaux alkylperfluorés en C6-C18 (par exemple le groupement de formule - (CH₂)₂-(CF₂)₉-CF₃) ; le radical cholestéryle (C₂₇) ou un reste d'ester de cholestérol comme le groupe oxyhexanoate de cholestéryle ; les groupes polycycliques aromatiques comme le naphtalène ou le pyrène. Parmi ces radicaux, on préfère plus particulièrement les radicaux alkyles linéaires et plus particulièrement le radical n-dodécyle.

Selon une forme particulièrement préférée de l'invention, le monomère de formule (IV) comporte au moins un motif oxyde d'alkylène (x ≥1) et de préférence une chaîne polyoxyalkylénée. La chaîne polyoxyalkylénée, de façon préférentielle, est constituée de motifs oxyde d'éthylène et/ou de motifs oxyde de propylène et encore plus particulièrement constituée de motifs oxyde d'éthylène. Le nombre de motifs oxyalkylénés varie en général de 3 à 100 et plus préférentiellement de 3 à 50 et encore plus préférentiellement de 7 à 25.

Parmi ces polymères, on peut citer :
- les copolymères réticulés ou non réticulés, neutralisés ou non, comportant de 15 à 60% en poids de motifs AMPS et de 40 à 85% en poids de motifs (C₈-C₁₆)alkyl(méth)acrylamide ou de motifs (C₈-C₁₆)alkyl(méth)acrylate par rapport au polymère, tels que ceux décrits dans la demande EP-A750 899 ;
- les terpolymères comportant de 10 à 90% en mole de motifs acrylamide, de 0,1 à 10% en mole de motifs AMPS et de 5 à 80% en mole de motifs n-(C₆-C₁₈)alkylacrylamide, tels que ceux décrits dans le brevet US- 5089578.

On peut également citer les copolymères d'AMPS totalement neutralisés et de méthacrylate de dodécyle ainsi que les copolymères d'AMPS et de n-dodécylméthacrylamide non-réticulés et réticulés, tels que ceux décrits dans les articles de Morishima cités ci-dessus.

On citera plus particulièrement les copolymères constitués de motifs acide 2-acrylamido-2-méthylpropane-sulfonique (AMPS) de formule (V) suivante : 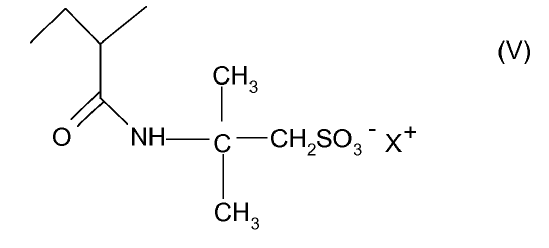 dans laquelle X+ est un proton, un cation de métal alcalin, un cation alcalino-terreux ou l'ion ammonium,
et de motifs de formule (VI) suivante : 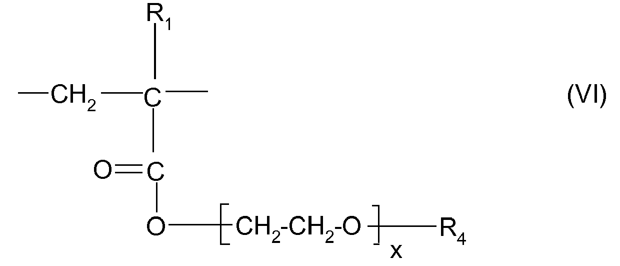 dans laquelle x désigne un nombre entier variant de 3 à 100, de préférence de 5 à 80 et plus préférentiellement de 7 à 25 ; R1 a la même signification que celle indiquée ci-dessus dans la formule (IV) et R4 désigne un alkyle linéaire ou ramifié en C6-C22 et plus préférentiellement en C10-C22.

Les polymères particulièrement préférés sont ceux pour lesquels x = 25, R1 désigne méthyle et R4 représente n-dodécyle ; ils sont décrits dans les articles de Morishima mentionnés ci-dessus.

Les polymères pour lesquels X+ désigne sodium ou ammonium sont plus particulièrement préférés.
(iii) Les polymères amphiphiles cationiques utilisés dans la présente invention sont choisis de préférence parmi les polyacrylates à groupements latéraux aminés.

Les polyacrylates à groupements latéraux aminés, quaternisés ou non, possèdent par exemple des groupements hydrophobes du type stéareth-20 (alcool stéarylique polyoxyéthyléné(20)) ou alkyl(C10-C30)PEG-20 itaconate.

Comme exemples de polyacrylates à chaînes latérales aminées, on peut citer les polymères 8781-124B ou 9492-103 ou STRUCTURE PLUS de la société NATIONAL STARCH.
(iv) A titre de polymères amphiphiles amphotère contenant au moins une chaîne grasse, on peut citer les copolymères de chlorure de méthacrylamidopropyl triméthylammonium/acide acrylique/méthacrylate d'alkyle en C10-C30, le radical alkyle étant de préférence un radical stéaryle.
   (b) Parmi les homopolymères d'acide acrylique réticulés, on peut citer ceux réticulés par un éther allylique d'alcool de la série du sucre, comme par exemple les produits vendus sous les noms CARBOPOLS 980, 981, 954, 2984 et 5984 par la société GOODRICH ou les produits vendus sous les noms SYNTHALEN M et SYNTHALEN K par la société 3 VSA.
   (c) Parmi les copolymères réticulés d'acide (méth)acrylique et d'acrylate d'alkyle en C1-C6, on peut citer le produit vendu sous la dénomination VISCOATEX 538C par la société COATEX qui est un copolymère réticulé d'acide méthacrylique et d'acrylate d'éthyle en dispersion aqueuse à 38% de Matière Active ou le produit vendu sous la dénomination ACULYN 33 par la société ROHM & HAAS qui est un copolymère réticulé d'acide acrylique et d'acrylate d'éthyle en dispersion aqueuse à 28% de Matière Active. On peut citer plus particulièrement le copolymère acide méthacrylique/acrylate d'éthyle réticulé sous forme de dispersion aqueuse à 30% fabriqué et vendu sous le nom CARBOPOL AQUA SF-1 par la société NOVEON.
   (d) Parmi les homopolymères ou copolymères non-ioniques contenant des monomères à insaturation éthylénique de type ester et/ou amide, on peut citer les produits vendus sous les dénominations de : CYANAMER P250 par la société CYTEC (polyacrylamide); PMMA MBX-8C par la société US COSMETICS (copolymère méthacrylate de méthyle/diméthacrylate d'éthylèneglycol); ACRYLOID B66 par la société RHOM & HAAS (copolymère méthacrylate de butyle/méthacrylate de méthyle); BPA 500 par la société KOBO (polyméthacrylate de méthyle).
   (e) Parmi les homopolymères d'acrylate d'ammonium, on peut citer le produit vendu sous le nom MICROSAP PAS 5193 par la société HOECHST.
      Parmi les copolymères d'acrylate d'ammonium et d'acrylamide, on peut citer le produit vendu sous le nom BOZEPOL C NOUVEAU ou le produit PAS 5193 vendus par la société HOECHST (ils sont décrits et préparés dans les documents FR-2416723, US-2798053 et US-2923692).
   (f) Acides poly(méth)acrylamido-alkyl(C1-C4)-sulfoniques

Selon la présente invention, le ou les acides poly(méth)acrylamido-alkyl(C1-C4)-sulfoniques sont de préférence réticulés.

Plus particulièrement encore, ils sont partiellement ou totalement neutralisés.

Ce sont des polymères hydrosolubles ou gonflables dans l'eau. Parmi ces polymères, on peut citer notamment :
- l'acide polyacrylamido-méthane-sulfonique,
- l'acide polyacrylamido-éthane-sulfonique,
- l'acide polyacrylamido-propane-sulfonique,
- l'acide poly-2-acrylamido-2-méthylpropane-sulfonique,
- l'acide poly-2-méthacrylamido-2-méthylpropane-sulfonique,
- l'acide poly-2-acrylamido-n-butane-sulfonique.

Des polymères de ce type et notamment des acides poly-2-acrylamido-2-méthylpropane-sulfoniques réticulés et partiellement ou totalement neutralisés sont connus, décrits et préparés dans la demande de brevet DE-19625810.

Ils sont en général caractérisés par le fait qu'ils comprennent, distribués de façon aléatoire :
a) de 90 à 99,9% en poids de motifs de formule (VII) suivante : 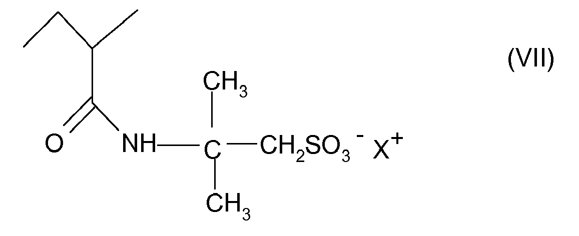 dans laquelle X + désigne un cation ou un mélange de cations, dont H+ ,
b) de 0,01 à 10% en poids d'au moins un motif réticulant ayant au moins deux doubles-liaisons oléfiniques,
   les proportions en poids étant définies par rapport au poids total du polymère;
   X+ représente un cation ou un mélange de cations choisis en particulier parmi un proton, un cation de métal alcalin, un cation équivalent de celui d'un métal alcalino-terreux ou l'ion ammonium.

De préférence, l'acide poly-2-acrylamido-2-méthylpropane-sulfonique réticulé et neutralisé comporte de 98 à 99,5 % en poids de motifs de formule (VII) et de 0,5 à 2 % en poids de motifs réticulants.

Les motifs réticulants ayant au moins deux doubles-liaisons oléfiniques sont choisis par exemple parmi le dipropylèneglycol-diallyléther, les polyglycol-diallyléthers, le triéthylèneglycol-divinyléther, l'hydroquinone-diallyl-éther, le tétra-allyl-oxyéthane ou d'autres allyl- ou vinyl-éthers d'alcools polyfonctionnels, le diacrylate de tétraéthylèneglycol, la triallylamine, le triméthylolpropane-diallyléther, le méthylène-bis-acrylamide ou le divinylbenzène.

Les motifs réticulants ayant au moins deux doubles-liaisons oléfiniques sont plus particulièrement encore choisis parmi ceux répondant à la formule générale (VIII) suivante : 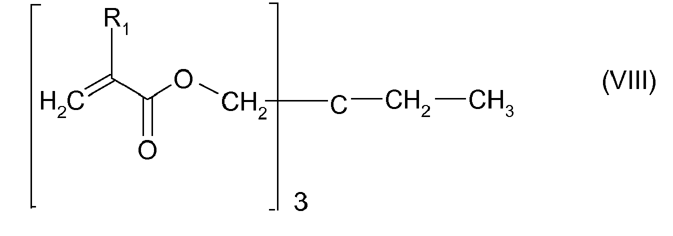 dans laquelle R1 désigne un atome d'hydrogène ou un alkyle en C1-C4 et plus particulièrement méthyle (triméthylol propane triacrylate).

Les acides poly-2-acrylamido-2-méthylpropane-sulfoniques réticulés et partiellement ou totalement neutralisés sont généralement connus sous les appellations "Ammonium Polycrylamido-2-methylpropanesulfonate" ou encore "Ammonium Polyacryldimethyltauramide" (appellation I.N.C.I.).

Un produit particulièrement préféré selon l'invention est celui vendu par la société CLARIANT sous la dénomination commerciale HOSTACERIN AMPS; c'est un acide poly-2-acrylamido-2-méthylpropane sulfonique réticulé et partiellement neutralisé par l'ammoniaque.
(g) Les polymères réticulés de sels de méthacryloyloxyalkyl(C1-C4) trialkyl(C1-C4)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de « SALCARE® SC 92 » par la Société CIBA. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de « SALCARE® SC 95 » et « SALCARE® SC 96 » par la Société CIBA.

De préférence, le ou les polymères épaississants (meth)acryliques selon l'invention sont anioniques.

La composition selon la présente invention comprend avantageusement de 0,05 à 20 %, de préférence de 0,1 à 10 % en poids d'un ou plusieurs polymères épaississants (méth)acryliques par rapport au poids total de la composition.

De préférence, le milieu cosmétiquement acceptable est aqueux.

La composition cosmétique selon l'invention peut comprendre en outre un ou plusieurs solvants organiques, de préférence dans une quantité comprise entre 0,05 et 40%, de manière tout à fait préférée, entre 1 et 20% en poids, par rapport au poids total de la composition.

Ce solvant organique peut être un alcool inférieur en C₂ à C₄, en particulier l'éthanol, les polyols et éthers de polyol tels que le propylène glycol, le polyéthylène glycol, le glycérol.

Les compositions selon l'invention peuvent également contenir d'autres adjuvants cosmétiquement acceptables, tels que par exemple des agents tensioactifs ioniques ou non-ioniques, des agents épaississants additionnels différents des polymères épaississants (méth)acryliques utilisés dans les compositions selon la présente demande, les alcools gras éthoxylés ou non, les agents co-épaississants, les agents de pénétration, les parfums, les colorants, les plastifiants, les tampons, et divers adjuvants usuels comme les cires, les silicones volatiles ou non, cycliques ou linéaires ou ramifiées, organomodifiées notamment alkoxylées ou modifiées par des groupements amines ou non modifiées par exemple les gommes de silicone, les céramides, les pseudocéramides, les huiles végétales, minérales ou de synthèse, les vitamines ou provitamines comme le panthénol, les opacifiants, les agents réducteurs, les émulsionnants, les conservateurs, les charges minérales, les nacres, les paillettes, les filtres solaires, les protéines, les polymères fixants anioniques, non ioniques, cationiques ou amphotères, les agents hydratants, les émollients, les agents adoucissants, les agents anti-mousse, les agents antiperspirants, les agents anti-radicaux libres, les bactéricides, les séquestrants, les anti-pelliculaires, les anti-oxydants, les agents alcalinisants, les agents acidifiants, et tout autre additif classiquement utilisé dans les compositions cosmétiques destinées à être appliquées sur les cheveux.

Les agents tensioactifs utilisables dans la composition selon la présente invention peuvent être des tensioactifs anioniques, non ioniques, amphotères ou cationiques, ou des mélanges de ceux-ci.

Parmi les tensioactifs anioniques utilisables, seuls ou en mélanges, dans le cadre de la présente invention, on peut notamment citer les sels, et en particulier les sels de métaux alcalins tels que les sels de sodium, les sels d'ammonium, les sels d'amines, les sels d'amino-alcools ou les sels de métaux alcalino-terreux, par exemple, de magnésium, des composés suivants : les alkylsulfates, les alkyléthersulfates, les alkylamidoéthersulfates, les alkylarylpolyéthersulfates, les monoglycéride-sulfates ; les alkylsulfonates, les alkylamidesulfonates, les alkyl-arylsulfonates, les α-oléfine-sulfonates, les paraffine-sulfonates ; les alkylsulfosuccinates, les alkyléthersulfosuccinates, les alkylamide-sulfosuccinates ; les alkylsulfoacétates ; les acylsarconisates ; et les acylglutamates, les groupes alkyle et acyle de tous ces composés comportant de 6 à 24 atomes de carbone et le groupe aryle désignant de préférence un groupe phényle ou benzyle.

On peut également utiliser dans le cadre de la présente invention les esters d'alkyle en C₆-C₂₄ et d'acides polyglycoside-carboxyliques tels que les glucoside-citrates d'alkyle, les polyglycoside-tartrates d'alkyle, et les polyglycoside-sulfosuccinates d'alkyle ; les alkylsulfosuccinamates, les acyliséthionates et les N-acyltaurates, le groupe alkyle ou acyle de tous ces composés comportant de 12 à 20 atomes de carbone. Parmi les tensioactifs anioniques encore utilisables, on peut également citer les acyllactylates dont le groupe acyle comporte de 8 à 20 atomes de carbone.

En outre, on peut encore citer les acides d'alkyl-D-galactoside uroniques et leurs sels ainsi que les acides alkyl(C₆-C₂₄)éther-carboxyliques polyoxyalkylénés, les acides alkyl(C₆-C₂₄)aryl(C₆-C₂₄)éther-carboxyliques polyoxyalkylénés, les acides alkyl(C₆-C₂₄)amidoéther carboxyliques polyoxyalkylénés et leurs sels, en particulier ceux comportant de 2 à 50 groupements oxyde d'éthylène, et leurs mélanges.

Les agents tensioactifs non-ioniques que l'on peut utiliser dans le cadre de la présente invention, sont eux aussi, des composés bien connus en soi (voir notamment à cet égard "Handbook of Surfactants" par M.R. PORTER, éditions Blackie & Son (Glasgow and London), 1991, pp 116-178) Ils peuvent être notamment choisis parmi les alcools, les alpha-diols, les alkyl(C₁-C₂₀) phénols ou les acides gras polyéthoxylés, polypropoxylés ou polyglycérolés, ayant une chaîne grasse comportant par exemple de 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et d'oxyde de propylène, les condensats d'oxyde d'éthylène et d'oxyde de propylène sur des alcools gras ; les amides gras polyéthoxylés ayant de préférence de 2 à 30 moles d'oxyde d'éthylène ; les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitane éthoxylés ayant de 2 à 30 moles d'oxyde d'éthylène ; les esters d'acides gras du saccharose, les esters d'acide gras du polyéthylèneglycol, les alkyl(C₆-C₂₄)polyglucosides, les dérivés de N-alkyl(C₆-C₂₄)glucamine, les oxydes d'amines tels que les oxydes d'alkyl(C₁₀-C₁₄)amines ou les oxydes de N-acyl(C₁₀-C₁₄)aminopropylmorpholine, et leurs mélanges.

Les agents tensioactifs amphotères, convenant dans la présente invention, peuvent être notamment des dérivés d'amines aliphatiques secondaires ou tertiaires, dans lesquels le groupe aliphatique est une chaîne linéaire ou ramifiée comportant 8 à 22 atomes de carbone et contenant, au moins un groupe anionique hydrosolubilisant tel que, par exemple, un groupe carboxylate, sulfonate, sulfate, phosphate ou phosphonate, on peut citer encore les alkyl(C₈-C₂₀)bétaïnes, les sulfobétaïnes, les alkyl(C₈-C₂₀)amidoalkyl(C₆-C₈)-bétaïnes ou les alkyl(C₈-C₁₀)amidoalkyl(C₆-C₈)sulfobétaïnes ; et leurs mélanges.

Parmi les dérivés d'amines, on peut citer les produits commercialisés sous la dénomination MIRANOL^{®}, tels que décrits dans les brevets US-2,528,378 et US-2,781,354 et classés dans le dictionnaire CTFA, 3^{ème} édition, 1982, sous les dénominations Amphocarboxy-glycinate et Amphocarboxypropionate de structures respectives (1) et (2) :

R₂ -CONHCH₂CH₂ -N⁺(R₃)(R₄)(CH₂COO⁻) (1)

dans laquelle :
R₂ désigne représente un groupe alkyle dérivé d'un acide R₂-COOH présent dans l'huile de coprah hydrolysée, un groupe heptyle, nonyle ou undécyle,
R₃ représente un groupe bêta-hydroxyéthyle, et
R₄ représente un groupe carboxyméthyle ;
Et

R₂ -CONHCH₂CH₂ -N(B)(C) (2)

dans laquelle :
B représente -CH₂CH₂OX',
C représente -(CH₂)_{z} -Y', avec z = 1 ou 2,
X' représente le groupement -CH₂CH₂-COOH ou un atome d'hydrogène,
Y' représente -COOH ou le groupe -CH₂ - CHOH - SO₃H,
R₂ représente le groupe alkyle d'un acide R_{2'}-COOH présent dans l'huile de coprah ou dans l'huile de lin hydrolysée, un groupe alkyle, notamment en C₁₇ et sa forme iso, un groupe C₁₇ insaturé.

Ces composés sont classés dans le dictionnaire CTFA, 5ème édition, 1993, sous les dénominations cocoamphodiacétate de disodium, lauroamphodiacétate de disodium, caprylamphodiacétate de disodium capryloamphodiacétate de disodium, cocoamphodipropionate de disodium, lauroamphodipropionate de disodium, caprylamphodipropionate de disodium, capryloamphodipropionate de disodium, acide lauroamphodipropionique, acide cocoamphodipropionique.

A titre d'exemple on peut citer le cocoamphodiacétate commercialisé sous la dénomination commerciale MIRANOL^{®} C2M concentré par la société RHODIA.

Parmi les tensioactifs amphothères, on utilise de préférence les alkyl(C₈-C₂₀)bétaïnes telles que la cocobétaïne, les alkyl(C₈-C₂₀)amidoalkyl(C₆-C₈)bétaïnes telles que la cocamidobétaïne, les alkylamphodiacétates comme le cocoamphodiacétate disodique, et leurs mélanges.

La composition selon l'invention peut comprendre en outre un ou plusieurs tensioactifs cationiques bien connus en soi, tels que les sels d'amines grasses primaires, secondaires ou tertiaires, éventuellement polyoxyalkylénées, les sels d'ammonium quaternaire tels que les chlorures ou les bromures de tétraalkylammonium, d'alkylamidoalkyltrialkylammonium, de trialkylbenzylammonium, de trialkylhydroxyalkylammonium ou d'alkylpyridinium, les dérivés d'imidazoline ; ou les oxydes d'amines à caractère cationique.

Les agents tensioactifs non ioniques, amphotères et cationiques décrits ci-dessus peuvent être utilisés seuls ou en mélanges et leur quantité est comprise entre 0,01% et 30% en poids, de préférence entre 0,05% et 20% en poids et mieux encore entre 0,1% et 10% en poids par rapport au poids total de la composition.

Les agents gélifiants et/ou épaississants additionnels différents des polymères épaississants (méth)acryliques et convenant pour les compositions de l'invention sont bien connus dans la technique et peuvent être choisis parmi les poly(oxyalkylène)glycols, les esters de poly(oxyalkylène)glycols, les alginates, les biosaccharides, les dérivés d'amidon, les gommes naturelles telles que les gommes de xanthane, de guar, de caroube, les scléroglucanes, les dérivés de chitine et de chitosane, les carraghénanes, les argiles, et leur mélanges.

A titre d'exemple d'agents gélifiants, notamment en phase aqueuse, on peut citer le FUCOGEL^{®} 1000 PP commercialisé par la société SOLABIA, le SATIAGEL^{®} KSO commercialisé par DEGUSSA et le KELTROL^{®} commercialisé par la société KELCO.

Les agents gélifiants additionnels représentent en général de 0,05 à 15%, de préférence de 0,5 à 10% en poids de la composition.

Les silicones utilisables en tant qu'additifs dans les compositions cosmétiques de la présente invention, sont des silicones volatiles ou non volatiles, cycliques, linéaires ou ramifiées, modifiées ou non par des groupements organiques, ayant une viscosité de 5.10⁻⁶ à 2,5m²/s à 25°C et de préférence 1.10⁻⁵ à 1m²/s.

Les silicones utilisables conformément à l'invention peuvent être solubles ou insolubles dans la composition et en particulier être des polyorganosiloxanes insolubles dans la composition de l'invention. Elles peuvent se présenter sous forme d'huiles, de cires, de résines ou de gommes.

Les organopolysiloxanes sont définis plus en détail dans l'ouvrage de Walter NOLL "Chemistry and Technology of Silicones" (1968), Academie Press. Elles peuvent être volatiles ou non volatiles.

Lorsqu'elles sont volatiles, les silicones sont plus particulièrement choisies parmi celles possédant un point d'ébullition compris entre 60°C et 260°C, et plus particulièrement encore parmi:
(i) les silicones cycliques comportant de 3 à 7, de préférence de 4 à 5 atomes de silicium. Il s'agit, par exemple, de l'octaméthylcyclotétrasiloxane commercialisé notamment sous le nom de VOLATILE SILICONE^{®} 7207 par UNION CARBIDE ou SILBIONE^{®} 70045 V2 par RHODIA, le décaméthylcyclopentasiloxane commercialisé sous le nom de VOLATILE SILICONE^{®} 7158 par UNION CARBIDE, et SILBIONE® 70045 V5 par RHODIA, ainsi que leurs mélanges.

On peut également citer les cyclocopolymères du type diméthylsiloxanes/ méthylalkylsiloxane, tel que la SILICONE VOLATILE^{®} FZ 3109 commercialisée par la société UNION CARBIDE, de formule :

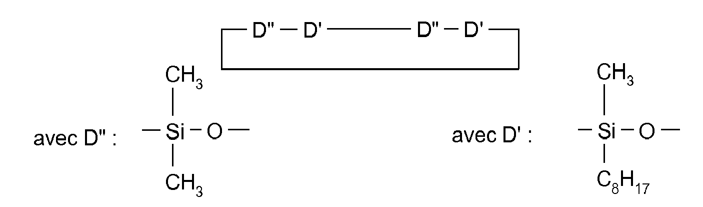

On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthylsilyloxy) bis-néopentane ;
(ii) les silicones volatiles linéaires ayant 2 à 9 atomes de silicium et présentant une viscosité inférieure ou égale à 5.10-6m²/s à 25°C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and Toiletries, Vol. 91, Jan. 76, P. 27-32 - TODD & BYERS "Volatile Silicone fluids for cosmetics".

On utilise de préférence des silicones non volatiles, et plus particulièrement des polyalkylsiloxanes, des polyarylsiloxanes, des polyalkylarylsiloxanes, des gommes et des résines de silicones, des polyorganosiloxanes modifiés par les groupements organofonctionnels ainsi que leurs mélanges.

Ces silicones sont plus particulièrement choisies parmi les polyalkylsiloxanes parmi lesquels on peut citer principalement les polydiméthylsiloxanes à groupements terminaux triméthylsilyl. La viscosité des silicones est mesurée à 25°C selon la norme ASTM 445 Appendice C.

Parmi ces polyalkylsiloxanes, on peut citer à titre non limitatif les produits commerciaux suivants :
- les huiles SILBIONE® des séries 47 et 70 047 ou les huiles MIRASIL® commercialisées par RHODIA telles que, par exemple l'huile 70 047 V 500 000 ;
- les huiles de la série MIRASIL® commercialisées par la société RHODIA ;
- les huiles de la série 200 de la société DOW CORNING telles que la DC200 ayant viscosité 60 000 mm²/s ;
- les huiles VISCASIL® de GENERAL ELECTRIC et certaines huiles des séries SF (SF 96, SF 18) de GENERAL ELECTRIC.

On peut également citer les polyméthylsiloxanes à groupements terminaux diméthylsilanol connus sous le nom de diméthiconol (CTFA), tels que les huiles de la série 48 de la société RHODIA.

Dans cette classe de polyalkylsiloxanes, on peut également citer les produits commercialisés sous les dénominations "ABIL WAX® 9800 et 9801" par la société GOLDSCHMIDT qui sont des polyalkyl (C₁-C₂₀) siloxanes.

Les polyalkylarylsiloxanes sont particulièrement choisis parmi les polydiméthyl/méthylphénylsiloxanes, les polydiméthyl/diphénylsiloxanes linéaires et/ou ramifiés de viscosité allant de 1.10⁻⁵ à 5.10⁻²m²/s à 25°C.

Parmi ces polyalkylarylsiloxanes on peut citer à titre d'exemple les produits commercialisés sous les dénominations suivantes :
- les huiles SILBIONE® de la série 70 641 de RHODIA;
- les huiles des séries RHODORSIL® 70 633 et 763 de RHODIA ;
- l'huile DOW CORNING 556 COSMETIC GRAD FLUID de DOW CORNING ;
- les silicones de la série PK de BAYER comme le produit PK20 ;
- les silicones des séries PN, PH de BAYER comme les produits PN1000 et PH1000 ;
- certaines huiles des séries SF de GENERAL ELECTRIC telles que SF 1023, SF 1154, SF 1250, SF 1265.

Les gommes de silicone utilisables conformément à l'invention sont notamment des polyorganosiloxanes, ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles polyphénylméthylsiloxanes (PPMS), les isoparaffines, les polyisobutylènes, le chlorure de méthylène, le pentane, le dodécane, le tridécane ou leurs mélanges.

On peut citer plus particulièrement les produits suivants :
- les gommes de polydiméthylsiloxane,
- les gommes de polydiméthylsiloxane/méthylvinylsiloxane,
- les gommes de polydiméthylsiloxane/diphénylsiloxane,
- les gommes de polydiméthylsiloxane/phénylméthylsiloxane,
- les gommes de polydiméthylsiloxane /diphénylsiloxane/méthylvinylsiloxane.

Des produits plus particulièrement utilisables conformément à l'invention sont des mélanges tels que :
- les mélanges formés à partir d'un polydiméthylsiloxane hydroxylé en bout de chaîne, ou diméthiconol (CTFA) et d'un polydiméthylsiloxane cyclique également appelé cyclométhicone (CTFA) tel que le produit Q2 1401 commercialisé par la société DOW CORNING ;
- les mélanges d'une gomme polydiméthylsiloxane et d'une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶m²/s. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les motifs :

R₂SiO_{2/2}, R₃SiO_{1/2}, RSiO_{3/2} et SiO_{4/2}

dans lesquelles R représente un groupement hydrocarboné possédant 1 à 16 atomes de carbone ou un groupement phényle. Parmi ces produits, ceux particulièrement préférés sont ceux dans lesquels R désigne un groupe alkyle inférieur en C₁-C₄, plus particulièrement méthyle ou un groupe phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organomodifiées utilisables conformément à l'invention sont des silicones telles que définies précédemment et comportant dans leur structure un ou plusieurs groupements organofonctionnels fixés par l'intermédiaire d'un groupe hydrocarboné.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone copolyol commercialisé par la société DOW CORNING sous la dénomination DC 1248 ou les huiles SILWET® L 722, L 7500, L 77, L 711 de la société UNION CARBIDE et l'alkyl (C₁₂)-méthicone copolyol commercialisée par la société DOW CORNING sous la dénomination Q2 5200 ;
- des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 929 ou 939, ou DOW CORNING 2-8299 par la société DOW CORNING ou le produit commercialisé sous la dénomination BELSIL ADM LOG 1 par la société WACKER. Les groupements aminés substitués sont en particulier des groupements aminoalkyles en C₁-C₄ ;
- des groupements thiols, comme les produits commercialisés sous les dénominations GP 72A et GP 71 de GENESEE.
- des groupements alcoxylés, comme le produit commercialisé sous la dénomination "SILICONE COPOLYMER F-755" par SWS SILICONES et ABIL WAX® 2428, 2434 et 2440 par la société GOLDSCHMIDT.
- des groupements hydroxylés, comme les polyorganosiloxanes à fonction hydroxyalkyle;
- des groupements alcoxyalkyle tels que, par exemple, les polyorganosiloxanes décrits dans le brevet US-A-4 957 732 ;
- des groupements anioniques du type carboxylique comme par exemple, dans le produits décrits dans le brevet EP 186 507 de la société CHISSO CORPORATION, ou de type alkyl-carboxyliques comme ceux présents dans le produit X-22-3701E de la société SHIN-ETSU ; 2-hydroxyalkylsulfonate ; 2-hydroxyalkylthiosulfate tels que les produits commercialisés par la société GOLDSCHMIDT sous les dénominations ABIL^{®} S201 ET ABIL^{®} S255 ;
- des groupements hydroxyacrylamino, comme les
polyorganosiloxanes décrits dans la demande EP 342 834. On peut citer, par exemple, le produit Q2-8413 de la société DOW CORNING.

Les silicones telles que décrites ci-dessus peuvent être utilisées seules ou en mélange, en une quantité comprise entre 0,01 et 20% en poids, de préférence entre 0,1 et 5% en poids.

Les compositions de l'invention peuvent encore comporter des corps gras non siliconés tels que les huiles minérales, végétales, animales et synthétiques, les cires, les esters gras, les alcools gras éthoxylés ou non éthoxylés, et les acides gras.

Comme huiles utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépin de raisin, de sésame, de noisette, d'abricot, de macadamia, d'arara, de ricin, d'avocat, les triglycérides des acides caprylique/caprique comme ceux vendus par la société Stearineries Dubois ou ceux vendus sous les dénominations Miglyol^{®} 810, 812 et 818 par la société Dynamit Nobel, l'huile de jojoba, l'huile de beurre de karité ;
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que Parléam^{®} ; les isoparaffines comme l'isohexadécane et l'isodécane.
- les huiles fluorées partiellement hydrocarbonées et/ou siliconées comme celles décrites dans le document JP-A-2-295912 ; comme huiles fluorées, on peut citer aussi le perfluorométhylcyclopentane et le perfluoro-1,3 diméthylcyclohexane, vendus sous les dénominations de "FLUTEC^{®} PC1" et "FLUTEC^{®} PC3" par la Société BNFL Fluorochemicals ; le perfluoro-1,2-diméthylcyclobutane ; les perfluoroalcanes tels que le dodécafluoropentane et le tétradécafluorohexane, vendus sous les dénominations de "PF 5050^{®}" et "PF 5060^{®}" par la Société 3M, ou encore le bromoperfluorooctyle vendu sous la dénomination "FORALKYL^{®}" par la Société Atochem ; le nonafluoro-méthoxybutane et le nonafluoroéthoxyisobutane ; les dérivés de perfluoromorpholine, tels que la 4-trifluorométhyl perfluoromorpholine vendue sous la dénomination "PF 5052^{®}" par la Société 3M ;

La cire ou les cires sont choisies notamment, parmi la cire de Carnauba, la cire de Candelila, et la cire d'Alfa, la cire de paraffine, l'ozokérite, les cires végétales comme la cire d'olivier, la cire de riz, la cire de jojoba hydrogénée ou les cires absolues de fleurs telles que la cire essentielle de fleur de cassis vendue par la société BERTIN (France), les cires animales comme les cires d'abeilles, ou les cires d'abeilles modifiées (cerabellina) ; d'autres cires ou matières premières cireuses utilisables selon l'invention sont notamment les cires marines telles que celle vendue par la Société SOPHIM sous la référence M82, les cires de polyéthylène ou de polyoléfines en général.

Les acides gras saturés ou insaturés sont choisis plus particulièrement parmi l'acide myristique, l'acide palmitique, l'acide stéarique, l'acide béhénique, l'acide oléïque, l'acide linoléïque, l'acide linolénique et l'acide isostéarique.

Les esters gras sont notamment les esters d'acides carboxyliques en particulier les esters mono, di, tri ou tétracarboxyliques.

Les esters d'acides carboxyliques sont notamment les esters d'acides aliphatiques saturés ou insaturés, linéaires ou ramifiés en C₁-C₂₆ et d'alcools aliphatiques saturés ou insaturés, linéaires ou ramifiés en C₁-C₂₆, le nombre total de carbone des esters étant supérieur ou égal à 10.

Parmi les monoesters, on peut citer le béhénate de dihydroabiétyle ; le béhénate d'octyldodécyle ; le béhénate d'isocétyle; le lactate de cétyle ; le lactate d'alkyle en C₁₂-C₁₅ ; le lactate d'isostéaryle ; le lactate de lauryle ; le lactate de linoléyle ; le lactate d'oléyle ; l'octanoate de (iso)stéaryle ; l'octanoate d'isocétyle ; l'octanoate d'octyle ; l'octanoate de cétyle ; l'oléate de décyle ; l'isostéarate d'isocétyle ; le laurate d'isocétyle ; le stéarate d'isocétyle; l'octanoate d'isodécyle ; l'oléate d'isodécyle ; l'isononanoate d'isononyle ; le palmitate d'isostéaryle ; le ricinoléate de méthyle acétyle ; le stéarate de myristyle ; l'isononanoate d'octyle ; l'isononate de 2-éthylhexyle ; le palmitate d'octyle ; le pélargonate d'octyle ; le stéarate d'octyle ; l'érucate d'octyldodécyle ; l'érucate d'oléyle ; les palmitates d'éthyle et d'isopropyle, le palmitate d'éthyl-2-héxyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le stéarate d'isobutyle ; le malate de dioctyle, le laurate d'hexyle, le laurate de 2-hexyldécyle.

On peut également utiliser les esters d'acides di ou tricarboxyliques en C₄-C₂₂ et d'alcools en C₁-C₂₂ et les esters d'acides mono di ou tricarboxyliques et d'alcools di, tri, tétra ou pentahydroxy en C₂-C₂₆.

On peut notamment citer : le sébacate de diéthyle ; le sébacate de diisopropyle ; l'adipate de diisopropyle ; l'adipate de di n-propyle ; l'adipate de dioctyle ; l'adipate de diisostéaryle ; le maléate de dioctyle ; l'undecylénate de glycéryle ; le stéarate d'octyldodécyl stéaroyl ; le monoricinoléate de pentaérythrityle ; le tétraisononanoate de pentaérythrityle ; le tétrapélargonate de pentaérythrityle ; le tétraisostéarate de pentaérythrityle ; le tétraoctanoate de pentaérythrityle ; le dicaprylate de propylène glycol ; le dicaprate de propylène glycol ; l'érucate de tridécyle ; le citrate de triisopropyle ; le citrate de triisotéaryle ; trilactate de glycéryle ; trioctanoate de glycéryle ; le citrate de trioctyldodécyle ; le citrate de trioléyle ; le dioctanoate de propylène glycol et le diheptanoate de néopentyl glycol. Les esters cités ci-dessus étant différents des esters de formule (I).

Parmi les esters cités ci-dessus, on préfère utiliser les palmitates d'éthyle et d'isopropyle, le palmitate d'éthyl-2-héxyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le stéarate d'isobutyle ; le malate de dioctyle, le laurate d'hexyle, le laurate de 2-hexyldécyle et l'isononanate d'isononyle, l'octanoate de cétyle.

Comme alcools gras on peut citer les alcools gras saturés ou insaturés, linéaires ou ramifiés ayant de 8 à 26 atomes de carbone, comme l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique.

Les corps gras représentent en général de 0,1 à 50%; de préférence 1 à 30%, et encore plus préférentiellement de 2 à 20% en poids de la composition totale.

Comme indiqué précédemment les compositions peuvent comprendre des polymères fixants différents des polymères utilisés selon l'invention. Par polymère fixant, on entend au sens de la présente invention tout polymère permettant de conférer une forme à la chevelure ou de maintenir la chevelure sous une forme donnée.

Tous les polymères fixants anioniques, cationiques, amphotères, non ioniques et leurs mélanges utilisés dans la technique peuvent être utilisés dans les compositions selon la présente demande.

Les polymères fixants peuvent être solubles dans le milieu cosmétiquement acceptable ou insolubles dans ce même milieu et utilisés dans ce cas sous forme de dispersions de particules solides ou liquides de polymère (latex ou pseudolatex).

Les polymères fixants anioniques généralement utilisés sont des polymères comportant des groupements dérivés d'acide carboxylique, sulfonique ou phosphorique et ont une masse moléculaire moyenne en nombre comprise entre environ 500 et 5 000 000.

Les polymères fixants anioniques à groupements carboxyliques préférés selon l'invention sont :
A) les copolymères d'acide acrylique et d'acrylamide vendus sous la forme de leurs sels de sodium sous les dénominations RETEN 421, 423 ou 425 par la Société HERCULES, les sels de sodium des acides polyhydroxycarboxyliques ;
B) Les copolymères d'acide acrylique ou méthacrylique avec un monomère monoéthylénique tel que l'éthylène, le styrène, les esters vinyliques, les esters d'acide acrylique ou méthacrylique, éventuellement greffés sur un polyalkylène-glycol tel que le polyéthylène-glycol, et éventuellement réticulés. De tels polymères sont décrits en particulier dans le brevet français n° 1 222 944 et la demande allemande n° 2 330 956, les copolymères de ce type comportant dans leur chaîne un motif acrylamide éventuellement N-alkylé et/ou hydroxyalkylé tels que décrits notamment dans les demandes de brevet luxembourgeois n^{os} 75370 et 75371 ou proposés sous la dénomination QUADRAMER par la société AMERICAN CYANAMID. On peut aussi citer les terpolymères acide acrylique /acrylate d'éthyle / N-tertio-butylacrylamide comme l'ULTRAHOLD STRONG vendu par la société BASF. On peut également citer les copolymères d'acide acrylique et de méthacrylate d'alkyle en C₁-C₄ et les terpolymères de vinylpyrrolidone, d'acide acrylique et de méthacrylate d'alkyle en C₁-C₂₀, par exemple, de lauryle, tels que celui commercialisé par la société ISP sous la dénomination ACRYLIDONE^{®} LM et les terpolymères acide méthacrylique/acrylate d'éthyle/acrylate de tertiobutyle tels que le produit commercialisé sous la dénomination LUVIMER^{®} 100 P par la société BASF.
   On peut aussi citer les copolymères acide méthacrylique/ acide acrylique/acrylate d'éthyle/méthacrylate de méthyle en dispersion aqueuse, commercialisé sous la dénomination AMERHOLD® DR 25 par la société AMERCHOL ;
C) Les copolymères d'acide crotonique, tels que ceux comportant dans leur chaîne des motifs acétate ou propionate de vinyle, et éventuellement d'autres monomères tels que les esters allylique ou méthallylique, éther vinylique ou ester vinylique d'un acide carboxylique saturé, linéaire ou ramifié, à longue chaîne hydrocarbonée, comme ceux comportant au moins 5 atomes de carbone, ces polymères pouvant éventuellement être greffés ou réticulés, ou encore un autre monomère ester vinylique, allylique ou méthallylique d'un acide carboxylique α- ou β-cyclique. De tels polymères sont décrits entre autres dans les brevets français n^{os} 1 222 944, 1 580 545, 2 265 782, 2 265 781, 1 564 110 et 2 439 798. Des produits commerciaux entrant dans cette classe sont les résines 28-29-30, 26-13-14 et 28-13-10 commercialisées par la société National Starch ;
D) Les copolymères d'acides ou d'anhydrides carboxyliques monoinsaturés en C₄-C₈ choisis parmi :
   - les copolymères comprenant (i) un ou plusieurs acides ou anhydrides maléique, fumarique, itaconique et (ii) au moins un monomère choisi parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées. De tels polymères sont décrits en particulier dans les brevets US nos 2 047 398, 2 723 248, 2 102 113, le brevet GB n° 839 805. Des produits commerciaux sont notamment ceux vendus sous les dénominations GANTREZ^{®} AN ou ES par la société ISP ;
   - les copolymères comprenant (i) un ou plusieurs motifs anhydrides maléique, citraconique, itaconique et (ii) un ou plusieurs monomères choisis parmi les esters allyliques ou méthallyliques comportant éventuellement un ou plusieurs groupements acrylamide, méthacrylamide, α-oléfine, esters acryliques ou méthacryliques, acides acrylique ou méthacrylique ou vinylpyrrolidone dans leur chaîne,
   les fonctions anhydrides de ces copolymères étant éventuellement monoestérifiées ou monoamidifiées.
   Ces polymères sont par exemple décrits dans les brevets français n^{os} 2 350 384 et 2 357 241 de la demanderesse ;
E) Les polyacrylamides comportant des groupements carboxylates.
F) Les homopolymères et copolymères comprenant des groupements sulfoniques comme les polymères comportant des motifs vinylsulfonique, styrène-sulfonique, naphtalène-sulfonique ou acrylamido-alkylsulfonique différents des polyesters sulfoniques ramifiés utilisés selon l'invention.

Ces polymères peuvent être notamment choisis parmi :
- les sels de l'acide polyvinylsulfonique ayant une masse moléculaire comprise entre environ 1 000 et 100 000, ainsi que les copolymères avec un comonomère insaturé tel que les acides acrylique ou méthacrylique et leurs esters, ainsi que l'acrylamide ou ses dérivés, les éthers vinyliques et la vinylpyrrolidone ;
- les sels de l'acide polystyrène-sulfonique tels que les sels de sodium vendus par exemple sous la dénomination Flexan^{®} 500 et Flexan^{®} 130 par National Starch. Ces composés sont décrits dans le brevet FR 2 198 719 ;
- les sels d'acides polyacrylamide-sulfoniques tels que ceux mentionnés dans le brevet US 4 128 631, et plus particulièrement l'acide polyacrylamidoéthylpropane-sulfonique vendu sous la dénomination COSMEDIA POLYMER HSP 1180 par HENKEL.

Comme autre polymère fixant anionique utilisable selon l'invention, on peut citer le polymère anionique séquencé branché vendu sous la dénomination Fixate G-100 L par la société LUBRIZOL.

Selon l'invention, les polymères fixants anioniques sont de préférence choisis parmi les copolymères d'acide acrylique ou des esters acryliques tels que les terpolymères acide acrylique/acrylate d'éthyle/N-tertiobutylacrylamide vendus notamment sous la dénomination ULTRAHOLD^{®} STRONG par la société BASF, les copolymères dérivés d'acide crotonique tels que les terpolymères acétate de vinyle/tertio-butylbenzoate de vinyle/acide crotonique et les terpolymères acide crotonique/acétate de vinyle/ néododécanoate de vinyle vendus notamment sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que les copolymères méthylvinyléther/anhydride maléique monoestérifié vendus, par exemple, sous la dénomination GANTREZ^{®} par la société ISP, les copolymères d'acide méthacrylique et de méthacrylate de méthyle vendus sous la dénomination EUDRAGIT^{®} L par la société ROHM PHARMA, les copolymères d'acide méthacrylique et d'acrylate d'éthyle vendus sous la dénomination LUVIMER^{®} MAEX ou MAE par la société BASF et les copolymères acétate de vinyle/acide crotonique vendus sous la dénomination LUVISET CA 66 par la société BASF et les copolymères acétate de vinyle/acide crotonique greffés par du polyéthylèneglycol vendus sous la dénomination ARISTOFLEX^{®} A par la société BASF, le polymère vendu sous la dénomination Fixate G-100 L par la société LUBRIZOL.

Les polymères filmogènes fixants cationiques utilisables selon la présente invention sont de préférence choisis parmi les polymères comportant des groupements amines primaires, secondaires, tertiaires et/ou quaternaires faisant partie de la chaîne polymère ou directement reliés à celle-ci, et ayant un poids moléculaire compris entre 500 et environ 5 000 000 et de préférence entre 1 000 et 3 000 000.

Parmi ces polymères, on peut citer plus particulièrement les polymères cationiques suivants:
(1) les homopolymères ou copolymères dérivés d'esters ou d'amides acryliques ou méthacryliques et comportant au moins un des motifs de formules suivantes: 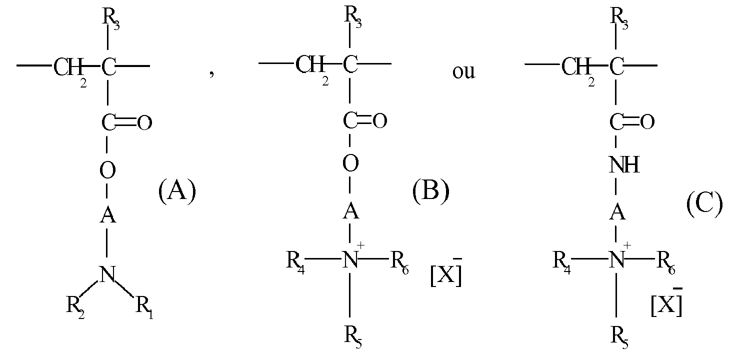 dans lesquelles :
   R3 désigne un atome d'hydrogène ou un radical CH3 ;
   A est un groupe alkyle linéaire ou ramifié comportant de 1 à 6 atomes de carbone ou un groupe hydroxyalkyle comportant de 1 à 4 atomes de carbone ;
   R4, R5, R6, identiques ou différents, représentent un groupe alkyle ayant de 1 à 18 atomes de carbone ou un radical benzyle ;
   R1 et R2, identiques ou différents, représentent chacun un atome hydrogène ou un groupe alkyle ayant de 1 à 6 atomes de carbone ;
   X désigne un anion méthosulfate ou un halogénure tel que chlorure ou bromure.
   Les copolymères de la famille (1) contiennent en outre un ou plusieurs motifs dérivant de comonomères pouvant être choisis dans la famille des acrylamides, méthacrylamides, diacétones-acrylamides, acrylamides et méthacrylamides substitués sur l'azote par des groupes alkyles inférieurs (C1-C4), des groupes dérivés des acides acryliques ou méthacryliques ou leurs esters, des vinyllactames tels que la vinylpyrrolidone ou le vinylcaprolactame, d'esters vinyliques.
   Ainsi, parmi ces copolymères de la famille (1), on peut citer :
   - les copolymères d'acrylamide et de méthacrylate de diméthylaminoéthyle quaternisé au sulfate de diméthyle ou avec un halogénure de diméthyle, tels que celui vendu sous la dénomination HERCOFLOC® par la société HERCULES,
   - les copolymères d'acrylamide et de chlorure de méthacryloyloxyéthyltriméthylammonium décrit par exemple dans la demande de brevet EP-A-080976 et vendus sous la dénomination BINA QUAT P 100 par la société CIBA GEIGY,
   - le copolymère d'acrylamide et de méthosulfate de méthacryloyloxyéthyltriméthylammonium tel que celui vendu sous la dénomination RETEN par la société HERCULES,
   - les copolymères vinylpyrrolidone/acrylate ou méthacrylate de dialkylaminoalkyle quaternisés ou non, tels que les produits vendus sous la dénomination "GAFQUAT® " par la société ISP comme par exemple "GAFQUAT® 734" ou "GAFQUAT® 755" ou bien les produits dénommés "COPOLYMER® 845, 958 et 937". Ces polymères sont décrits en détail dans les brevets français 2.077.143 et 2.393.573,
   - les polymères à chaîne grasse et à motif vinylpyrrolidone, tels que les produits vendus sous la dénomination Stylèze W20 et Stylèze W10 par la société ISP,
   - les terpolymères méthacrylate de diméthylaminoéthyle/vinylcaprolactame/vinylpyrrolidone tel que le produit commercialisé sous la dénomination GAFFIX VC 713 par la société ISP, et
   - les copolymères vinylpyrrolidone/méthacrylamide de diméthylaminopropyle quaternisés tels que les produits commercialisés sous la dénomination "GAFQUAT® HS 100" par la société ISP
(2) les polysaccharides cationiques non cellulosiques, de préférence à ammonium quaternaire tels que ceux décrits dans les brevets américains 3.589.578 et 4.031.307 tel que les gommes de guar contenant des groupements cationiques trialkylammonium. De tels produits sont commercialisés notamment sous les dénominations commerciales de JAGUAR C13 S, JAGUAR C 15, JAGUAR C 17 par la société MEYHALL.
(3) les copolymères quaternaires de vinylpyrrolidone et de vinylimidazole ;
(4) les chitosanes ou leurs sels; les sels utilisables sont en particulier les acétate, lactate, glutamate, gluconate ou le pyrrolidone-carboxylate de chitosane .
   Parmi ces composés, on peut citer le chitosane ayant un taux de désacétylation de 90,5% en poids vendu sous la dénomination KYTAN BRUT STANDARD par la société ABER TECHNOLOGIES, le pyrrolidone-carboxylate de chitosane commercialisé sous la dénomination KYTAMER® PC par la société AMERCHOL .
(5) les dérivés de cellulose cationiques tels que les copolymères de cellulose ou de dérivés de cellulose greffés avec un monomère hydrosoluble comportant un ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyloxyéthyl triméthylammonium, de méthacrylamidopropyl triméthylammonium, de diméthyl-diallylammonium.

Les produits commercialisés répondant à cette définition sont plus particulièrement les produits vendus sous la dénomination "CELQUAT L 200" et "CELQUAT H 100" par la Société National Starch.

Les polymères fixants amphotères utilisables conformément à l'invention peuvent être choisis parmi les polymères comportant des motifs B et C répartis statistiquement dans la chaîne polymère où B désigne un motif dérivant d'un monomère comportant au moins un atome d'azote basique et C désigne un motif dérivant d'un monomère acide comportant un ou plusieurs groupements carboxyliques ou sulfoniques, ou bien B et C peuvent désigner des groupements dérivant de monomères zwittérioniques de carboxybétaïnes ou de sulfobétaïnes;

B et C peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un groupe hydrocarboné, ou bien B et C font partie d'une chaîne d'un polymère à motif éthylène-α,β-dicarboxylique dont l'un des groupements carboxyliques a été amené à réagir avec une polyamine comportant un ou plusieurs groupements amines primaires ou secondaires.

Les polymères fixants amphotères répondant à la définition donnée ci-dessus plus particulièrement préférés sont choisis parmi les polymères suivants :
(1) les copolymères à motifs vinyliques acides et à motifs vinyliques basiques, tels que ceux résultant de la copolymérisation d'un monomère dérivé d'un composé vinylique portant un groupement carboxylique tel que plus particulièrement l'acide acrylique, l'acide méthacrylique, l'acide maléïque, l'acide alpha-chloracrylique, et d'un monomère basique dérivé d'un composé vinylique substitué contenant au moins un atome basique, tel que plus particulièrement les méthacrylate et acrylate de dialkylaminoalkyle, les dialkylaminoalkyl-méthacrylamide et acrylamide. De tels composés sont décrits dans le brevet américain n° 3 836 537.
(2) les polymères comportant des motifs dérivant :
   a) d'au moins un monomère choisi parmi les acrylamides ou les méthacrylamides substitués sur l'atome d'azote par un groupe alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique, et le produit de quaternisation du méthacrylate de diméthylaminoéthyle avec le sulfate de diméthyle ou diéthyle.
      Les acrylamides ou méthacrylamides N-substitués plus particulièrement préférés selon l'invention sont les composés dont les groupes alkyles comportent de 2 à 12 atomes de carbone, et plus particulièrement le N-éthylacrylamide, le N-tertiobutylacrylamide, le N-tertiooctylacrylamide, le N-octylacrylamide, le N-décylacrylamide, le N-dodécylacrylamide ainsi que les méthacrylamides correspondants.
      Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléïque ou fumarique.
      Les comonomères basiques préférés sont des méthacrylates d'aminoéthyle, de butylaminoéthyle, de N,N'-diméthylaminoéthyle, de N-tertio-butylaminoéthyle.
      On utilise particulièrement les copolymères dont la dénomination CTFA (4ème Ed., 1991) est Octylacrylamide/acrylates/butylaminoethyl-methacrylate copolymer, tels que les produits vendus sous la dénomination AMPHOMER^{®} ou LOVOCRYL^{®} 47 par la société NATIONAL STARCH.
      (3) les polyaminoamides réticulés et acylés partiellement ou totalement dérivant de polyaminoamides de formule générale : 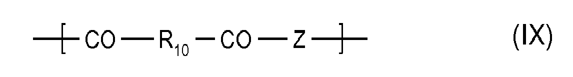 dans laquelle R₁₀ représente un groupe divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un groupe dérivant de l'addition de l'un quelconque desdits acides avec une amine bis-primaire ou bis-secondaire, et Z désigne un groupe dérivant d'une polyalkylène-polyamine bis-primaire, mono- ou bis-secondaire et de préférence représente :
         a) dans les proportions de 60 à 100 % en moles, le groupe 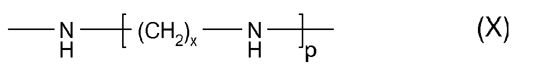 où x=2 et p=2 ou 3, ou bien x=3 et p=2
            ce groupe dérivant de la diéthylène-triamine, de la triéthylène-tétraamine ou de la dipropylène-triamine;
         b) dans les proportions de 0 à 40 % en moles, le groupe (X) ci-dessus, dans lequel x=2 et p=1 et qui dérive de l'éthylène-diamine, ou le groupe dérivant de la pipérazine : 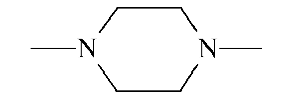
         c) dans les proportions de 0 à 20 % en moles, le groupe -NH-(CH₂)₆-NH- dérivant de l'hexaméthylènediamine,
            ces polyaminoamides étant réticulés par réaction d'addition d'un agent réticulant bifonctionnel choisi parmi les épihalohydrines, les diépoxydes, les dianhydrides, les dérivés bis-insaturés, au moyen de 0,025 à 0,35 mole d'agent réticulant par groupement amine du polyaminoamide, et acylés par action d'acide acrylique, d'acide chloracétique ou d'une alcane-sultone ou de leurs sels.
      Les acides carboxyliques saturés sont choisis de préférence parmi les acides ayant 6 à 10 atomes de carbone tels que les acides adipique, triméthyl-2,2,4-adipique et triméthyl-2,4,4-adipique, téréphtalique, les acides à double liaison éthylénique comme, par exemple, les acides acrylique, méthacrylique, itaconique.
      Les alcane-sultones utilisées dans l'acylation sont de préférence la propane- ou la butane-sultone, les sels des agents d'acylation sont de préférence les sels de sodium ou de potassium.
(4) les polymères comportant des motifs zwittérioniques de formule : 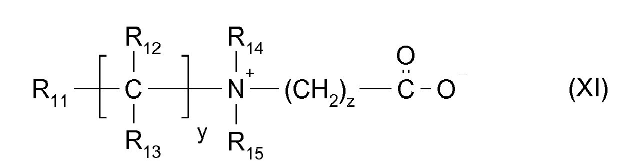 dans laquelle R₁₁ désigne un groupement insaturé polymérisable tel qu'un groupement acrylate, méthacrylate, acrylamide ou méthacrylamide, y et z représentent un nombre entier de 1 à 3, R₁₂ et R₁₃ représentent un atome d'hydrogène, un groupe méthyle, éthyle ou propyle, R₁₄ et R₁₅ représentent un atome d'hydrogène ou un groupe alkyle de telle façon que la somme des atomes de carbone dans R₁₄ et R₁₅ ne dépasse pas 10.
   Les polymères comprenant de tels motifs peuvent également comporter des motifs dérivés de monomères non zwittérioniques tels que l'acrylate ou le méthacrylate de diméthyl- ou diéthylaminoéthyle ou des acrylates ou méthacrylates d'alkyle, des acrylamides ou méthacrylamides, ou l'acétate de vinyle.
   A titre d'exemple, on peut citer les copolymères méthacrylate de méthyle/diméthyl-carboxyméthylammonio-éthylméthacrylate de méthyle, tel que le produit vendu sous la dénomination DIAFORMER Z301 par la société SANDOZ.
(5) les polymères dérivés du chitosane comportant des motifs monomères répondant aux formules suivantes : 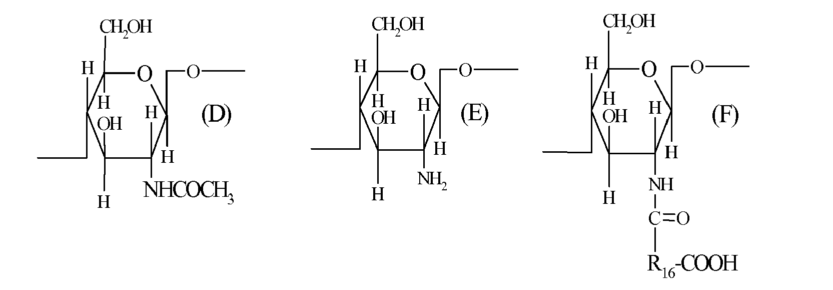 le motif (D) étant présent dans des proportions comprises entre 0 et 30%, le motif (E) dans des proportions comprises entre 5 et 50% et le motif (F) dans des proportions comprises entre 30 et 90%, étant entendu que dans ce motif (F), R₁₆ représente un groupe de formule : 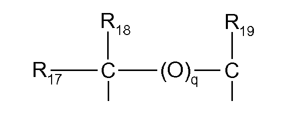 dans laquelle si q=0, R₁₇, R₁₈ et R₁₉, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des groupes R₁₇, R₁₈ et R₁₉ étant dans ce cas un atome d'hydrogène ;
   ou si q=1, R₁₇, R₁₈ et R₁₉ représentent chacun un atome d'hydrogène, ainsi que les sels formés par ces composés avec des bases ou des acides.
(6) Les polymères aux motifs répondant à la formule générale (XII) sont, par exemple, décrits dans le brevet français 1 400 366 : 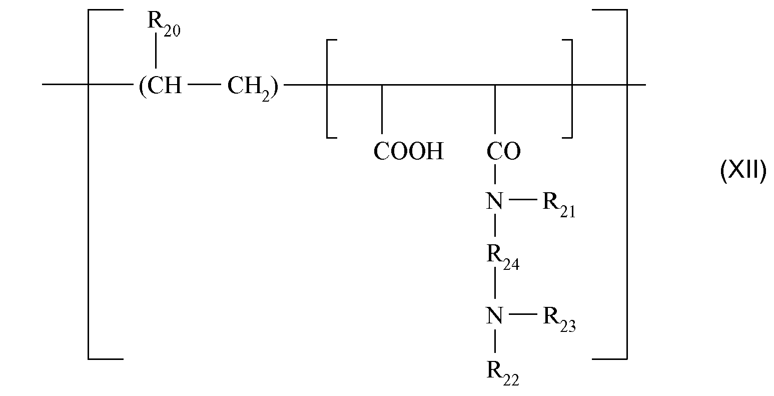 dans laquelle R₂₀ représente un atome d'hydrogène, un groupe CH₃O, CH₃CH₂O, phényle, R₂₁ désigne un atome d'hydrogène ou un groupe alkyle inférieur tel que méthyle, éthyle, R₂₂ désigne un atome d'hydrogène ou un groupe alkyle inférieur en C₁-C₆ tel que méthyle, éthyle, R₂₃ désigne un groupe alkyle inférieur en C₁-C₆ tel que méthyle, éthyle ou un groupe répondant à la formule : -R₂₄-N(R₂₂)₂, R₂₄ représentant un groupement -CH₂-CH₂-, -CH₂-CH₂-CH₂-, -CH₂-CH(CH₃)-, R₂₂ ayant les significations mentionnées ci-dessus.
(7) Les polymères dérivés de la N-carboxyalkylation du chitosane comme le N-carboxyméthyl-chitosane ou le N-carboxybutyl-chitosane, vendus sous la dénomination « EVALSAN » par la société JAN DEKKER.
(8) Les polymères amphotères du type -D-X-D-X choisis parmi :
   a) les polymères obtenus par action de l'acide chloracétique ou le chloracétate de sodium sur les composés comportant au moins un motif de formule :

      -D-X-D-X-D- (XIII)

      où D désigne un groupe 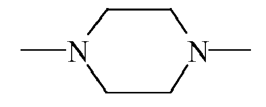 et X désigne le symbole E ou E', E ou E' identiques ou différents désignent un groupe bivalent qui est un groupe alkylène à chaîne droite ou ramifiée, comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques ; les atomes d'oxygène, d'azote et de soufre étant présents sous forme de groupements éther, thioéther, sulfoxyde, sulfone, sulfonium, alkylamine, alcénylamine, des groupements hydroxyle, benzylamine, oxyde d'amine, ammonium quaternaire, amide, imide, alcool, ester et/ou uréthanne.
   b) Les polymères de formule :

      -D-X-D-X- (XIV)

      où D désigne un groupe 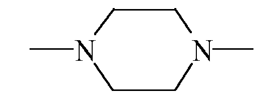 et X désigne le symbole E ou E' et au moins une fois E' ; E ayant la signification indiquée ci-dessus et E' est un groupe bivalent qui est un groupe alkylène à chaîne droite ou ramifiée, ayant jusqu'à 7 atomes de carbone dans la chaîne principale, substitué ou non par un ou plusieurs groupes hydroxyles et comportant un ou plusieurs atomes d'azote, l'atome d'azote étant substitué par une chaîne alkyle interrompue éventuellement par un atome d'oxygène et comportant obligatoirement une ou plusieurs fonctions carboxyles ou une ou plusieurs fonctions hydroxyles et bétaïnisées par réaction avec l'acide chloracétique ou du chloracétate de soude.
(9) les copolymères alkyl(C₁-C₅)vinyléther/anhydride maléique modifiés partiellement par semiamidification avec une N,N-dialkylaminoalkylamine telle que la N,N-diméthylaminopropylamine ou par semiestérification avec un N,N-dialkylaminoalcanol. Ces copolymères peuvent également comporter d'autres comonomères vinyliques tels que le vinylcaprolactame.

Parmi les polymères fixants amphotères cités ci-dessus les plus particulièrement préférés selon l'invention, on citera ceux de la famille (3) tels que les copolymères dont la dénomination CTFA est Octylacrylamide/acrylates/butylamino-ethylmethacrylate copolymer, tels que les produits vendus sous les dénominations AMPHOMER^{®}, AMPHOMER^{®} LV 71 ou LOVOCRYL^{®} 47 par la société NATIONAL STARCH et ceux de la famille (4) tels que les copolymères méthacrylate de méthyle/diméthyl-carboxyméthylammonio-éthylméthacrylate de méthyle vendu par exemple sous la dénomination DIAFORMER Z301 par la société SANDOZ.

Les polymères fixants non ioniques utilisables selon la présente invention sont choisis, par exemple, parmi :
- les polyalkyloxazolines ;
- les homopolymères d'acétate de vinyle ;
- les copolymères d'acétate de vinyle tels que, par exemple, les copolymères d'acétate de vinyle et d'ester acrylique, les copolymères d'acétate de vinyle et d'éthylène, ou les copolymères d'acétate de vinyle et d'ester maléïque, par exemple, de maléate de dibutyle ;
- les homopolymères et copolymères d'esters acryliques tels que, par exemple, les copolymères d'acrylates d'alkyle et de méthacrylates d'alkyle tels que les produits proposés par la société ROHM & HAAS sous les dénominations PRIMAL^{®} AC-261 K et EUDRAGIT^{®} NE 30 D, par la société BASF sous la dénomination 8845, par la société HOECHST sous la dénomination APPRETAN^{®} N9212 ;
- les copolymères d'acrylonitrile et d'un monomère non ionique choisis, par exemple, parmi le butadiène et les (méth)acrylates d'alkyle ; on peut citer les produits proposés sous la dénomination CJ 0601 B par la société ROHM & HAAS ;
- les homopolymères de styrène ;
- les copolymères de styrène comme, par exemple, les copolymères de styrène et de (méth)acrylate d'alkyle tels que les produits MOWILITH^{®} LDM 6911, MOWILITH^{®} DM 611 et MOWILITH^{®} LDM 6070 proposés par la société HOECHST, les produits RHODOPAS^{®} SD 215 et RHODOPAS^{®} DS 910 proposés par la société RHONE POULENC ; les copolymères de styrène, de méthacrylate d'alkyle et d'acrylate d'alkyle ; les copolymères de styrène et de butadiène ; ou les copolymères de styrène, de butadiène et de vinylpyridine ;
- les polyamides ;
- les homopolymères de vinyllactame tels que les homopolymères de vinylpyrrolidone et tels que le polyvinylcaprolactame commercialisé sous la dénomination Luviskol^{®} PLUS par la société BASF ; et
- les copolymères de vinyllactame tels qu'un copolymère poly(vinylpyrrolidone/vinyllactame) vendu sous le nom commercial Luvitec^{®} VPC 55K65W par la société BASF, les copolymères poly(vinylpyrrolidone/acétate de vinyle) comme ceux commercialisés sous la dénomination PVPVA^{®} S630L par la société ISP, Luviskol^{®} VA 73, VA 64, VA 55, VA 37 et VA 28 par la société BASF ; et les terpolymères poly(vinylpyrrolidone/acétate de vinyle/propionate de vinyle) comme, par exemple, celui commercialisé sous la dénomination Luviskol^{®} VAP 343 par la société BASF.

Les groupes alkyles des polymères non ioniques mentionnés ci-dessus ont, de préférence, de 1 à 6 atomes de carbone.

Selon l'invention, on peut également utiliser des polymères fixants de type siliconés greffés, comprenant une partie polysiloxane et une partie constituée d'une chaîne organique non siliconée, l'une des deux parties constituant la chaîne principale du polymère et l'autre étant greffée sur ladite chaîne principale.

Ces polymères sont par exemple décrits dans les demandes de brevet EP-A-0 412 704, EP-A-0 412 707, EP-A-0 640 105 et WO 95/00578, EP-A-0 582 152 et WO 93/23009 et les brevets US 4,693,935, US 4,728,571 et US 4,972,037.

Ces polymères peuvent être amphotères, anioniques ou non ioniques, et ils sont de préférence anioniques ou non ioniques.

De tels polymères sont, par exemple, les copolymères susceptibles d'être obtenus par polymérisation radicalaire à partir du mélange de monomères formé :
a) de 50 à 90 % en poids d'acrylate de tertiobutyle,
b) de 0 à 40 % en poids d'acide acrylique,
c) de 5 à 40 % en poids d'un macromère siliconé de formule 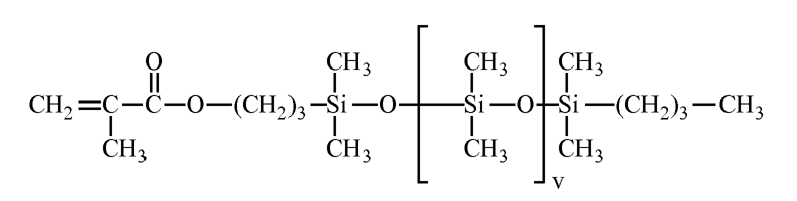
où v est un nombre allant de 5 à 700, les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type poly(acide (méth)acrylique) et du type poly((méth)acrylate d'alkyle), et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

Comme autre type de polymères fixants siliconés, on peut citer le produit Luviflex^{®} Silk commercialisé par la société BASF.

On peut également utiliser comme polymères fixants, des polyuréthanes fonctionnalisés ou non, siliconés ou non, cationiques, non-ioniques, anioniques ou amphotères, ou leurs mélanges.

Les polyuréthanes particulièrement visés par la présente invention sont ceux décrits dans les demandes EP 0 751 162, EP 0 637 600, EP 0 648 485 et FR 2 743 297 dont la demanderesse est titulaire, ainsi que dans les demandes EP 0 656 021 et WO 94/03510 de la société BASF, et EP 0 619 111 de la société National Starch.

Comme polyuréthanes convenant particulièrement bien dans la présente invention, on peut citer les produits commercialisés sous les dénominations LUVISET PUR^{®} et LUVISET^{®} Si PUR par la société BASF.

La concentration en polymère(s) fixant(s) utilisée dans les compositions selon la présente invention est comprise entre 0,1 et 20%, de préférence entre 0,5 et 10% en poids par rapport au poids total de la composition.

L'homme du métier saurait ajouter les additifs sans perturber les propriétés des compositions de l'invention.

De préférence, les compositions se présentent sous forme de gels. De préférence les compositions ont une viscosité supérieure à 500 cps à la température de 25°C et à un taux de cisaillement de 1s⁻¹.

Lorsque la composition selon l'invention est conditionnée dans un dispositif aérosol, elle comprend au moins un agent propulseur, qui peut être choisi parmi les hydrocarbures volatiles, tels que le N-butane, le propane, l'isobutane, le pentane, les hydrocarbures halogénés et leurs mélanges. On peut également utiliser en tant que propulseur le gaz carbonique, le protoxyde d'azote, le diméthyléther (DME), l'azote, ou l'air comprimé. On peut aussi utiliser des mélanges de propulseurs. De préférence, on utilise le diméthyléther.

Avantageusement, l'agent propulseur est présent à une concentration comprise entre 5 et 90% en poids par rapport au poids total de la composition dans le dispositif aérosol, et, plus particulièrement, à une concentration comprise entre 10 et 60%.

La composition selon l'invention peut notamment être utilisée en application non rincée sur les cheveux.

L'invention a également pour objet un procédé de mise en forme des cheveux, comprenant l'application d'une composition cosmétique selon l'invention. En particulier, l'invention concerne un procédé de coiffage comprenant l'application d'une composition selon l'invention sur les cheveux, le rinçage éventuel des cheveux, puis la mise en forme et le séchage des cheveux.

Les exemples qui suivent illustrent l'invention sans en limiter la portée.

### Exemples

Les compositions suivantes ont été réalisées :

Les concentrations sont exprimées en gramme de matières actives pour 100 grammes de composition.

### Exemples de gels

| | Composition | Composition | Composition | Composition |
|---|---|---|---|---|
| | A | B | C | D |
| Polyester sulfonique ramifié (1) | 1 | 2 | 3 | 3,8 |
| Copolymère VP/VA (2) | 3 | - | - | - |
| Polymère réticulé d'acrylates et d'acrylate d'alkyle en C10-C30 (3) | 1,65 | - | - | - |
| polyacrylate de glycéryle (4) | - | - | - | 0,15 |
| gomme de guar hydroxypropylée | - | 0,35 | - | - |
| Carbopol 980 (5) | - | 0,17 | - | - |
| taurate de polyacryloyldimethyl ammonium (6) | - | - | 1,5 | - |
| Polyacrylate-3 (7) | - | - | - | 0,8 |
| Copolymère acrylates-AMP/allylméthacrylates (8) | - | 2,8 | - | - |
| Copolymère VP/ diméthylamino éthyl méthacrylate (9) | 1,5 | - | - | - |
| parfum | 0,4 | 0,4 | 0,5 | 0,5 |
| Huile de ricin hydrogénée PEG-40(10) | 0,8 | 0,8 | 1 | 1 |
| Pentetate de pentasodium | - | 0,05 | 0,1 | - |
| triethanolamine | 2 | 1,5 | - | 0,22 |
| conservateur | 1 | 0,95 | 0,35 | 0,95 |
| sorbitol | - | - | 2,7 | - |
| PEG-8 | - | - | - | 2,2 |
| glycerol | 3 | - | 1,8 | 1,8 |
| Propylène glycol | 3 | 3 | 2,5 | - |
| Alcool dénaturé | 2,8 | - | - | - |
| Eau | Qsp 100 | Qsp 100 | Qsp 100 | Qsp 100 |

| | | | | |
|---|---|---|---|---|
| (1) Eastman AQ 1350® (2) copolymère vinylpyrrolidone/acétate de vinyle LUVISKOL® VA 64P de BASF (3) Pemulen TR-1 (Lubrizol) (4) Hispagel 200 (Cognis) (5) acide polyacrylique réticulé vendu par LUBRIZOL (6) Hostacerin AMPS (Clariant) (7) Viscophobe DB1000 (Dow Chemical) (8) Fixate G100L (Lubrizol) (9) Copolymere 845 (ISP) (10) CREMOPHOR CO 40 (BASF) | | | | |

Les compositions ci-dessus présentent une consistance adéquate permettant une application facile et une localisation aisée sur des cheveux propres. Après séchage la chevelure présente un maintien naturel de longue durée.

## Revendications

1. Composition cosmétique **caractérisée en ce qu'**elle comprend, dans un milieu cosmétiquement acceptable :
(i) un ou plusieurs polyesters sulfoniques ramifiés,
(ii) un ou plusieurs polymères épaississants (méth)acryliques et
(iii) un ou plusieurs tensioactifs choisis parmi les tensioactifs anioniques, non ioniques, amphotères ou cationiques.

2. Composition cosmétique selon la revendication 1 dans laquelle le polyester sulfonique ramifié est obtenu par polycondensation :
(a) d'au moins un acide dicarboxylique ne portant pas de fonction sulfonique,
(b) d'au moins un diol ou d'un mélange d'un diol et d'une diamine,
(c) d'au moins un monomère comportant deux fonctions réactives, identiques ou différentes, choisies parmi les groupes hydroxyle, amino et carboxyle, et portant en outre au moins une fonction sulfonique, et
(d) d'au moins un monomère comportant au moins trois fonctions réactives, identiques ou différentes, choisies parmi les groupes hydroxyle, amino et carboxyle.

3. Composition cosmétique selon la revendication 2, dans laquelle le polyester sulfonique ramifié comporte des motifs (e) dérivés de monomères comportant deux fonctions réactives différentes, choisis parmi les hydroxyacides carboxyliques et les aminoacides carboxyliques ou les mélange de ceux-ci .

4. Composition cosmétique selon l'une quelconque des revendications précédentes comprenant de 0,2 à 15%, de préférence de 0,5 à 10% et de manière encore préférée de 1 à 10% en poids d'un ou plusieurs polyesters sulfoniques ramifiés par rapport au poids total de la composition .

5. Composition cosmétique selon l'une quelconque des revendications précédentes dans laquelle le ou les polymères épaississants (méth)acryliques sont choisis parmi :
- les épaississants associatifs acryliques ;
- les homopolymères d'acide acrylique réticulés ;
- les copolymères réticulés d'acide (méth)acrylique et d'acrylate d'alkyle(C₁-C₆) ;
- les homopolymères et copolymères non-ioniques contenant des monomères à insaturation éthylénique de type ester et/ou amide ;
- les homopolymères d'acrylate d'ammonium ou les copolymères d'acrylate d'ammonium et d'acrylamide ;
- les homopolymères et copolymères d'acides (méth)acrylamido alkyl(C₁-C₄) sulfoniques ;
- les homopolymères et copolymères de méthacryoyl alkyl(C₁-C₄) trialkyl(C₁-C₄) ammonium réticulés.

6. Composition cosmétique selon l'une quelconque des revendications précédentes dans laquelle le ou les polymères épaississants (méth)acryliques sont anioniques.

7. Composition cosmétique selon l'une quelconque des revendications précédente comprenant de 0,05 à 20 %, de préférence de 0,1 à 10 % en poids d'un ou plusieurs polymères épaississants (méth)acryliques par rapport au poids total de la composition.

8. Composition cosmétique selon l'une quelconque des revendications précédentes comprenant un ou plusieurs gélifiants et/ou épaississants additionnels choisis parmi les poly(oxyalkylène)glycols, les esters de poly(oxyalkylène)glycols, les alginates, les biosaccharides, les dérivés d'amidon, les gommes naturelles telles que les gommes de xanthane, de guar, de caroube, les scléroglucanes, les dérivés de chitine et de chitosane, les carraghénanes, les argiles, et leur mélanges.

9. Composition cosmétique selon l'une quelconque des revendications précédentes comprenant un ou plusieurs composés choisis parmi les silicones et les corps gras non siliconés choisis parmi les huiles minérales, végétales, animales et synthétiques, les cires, les esters gras, les alcools gras éthoxylés ou non éthoxylés, et les acides gras.

10. Composition cosmétique selon l'une quelconque des revendications précédentes comprenant un ou plusieurs polymères fixants choisis parmi les polymères fixants anioniques, non ioniques, amphotères ou cationiques.

11. Utilisation de la composition cosmétique selon l'une quelconque des revendications précédentes pour le coiffage notamment pour le coiffage ou la mise en forme des matières kératiniques, de préférence les matières kératiniques humaines et en particulier les cheveux.

12. Procédé de coiffage comprenant l'application d'une composition selon l'une quelconque des revendications 1 à 10 sur les cheveux, le rinçage éventuel des cheveux, puis la mise en forme et le séchage des cheveux.
